## Europäisches Patentamt
## European Patent Office
## Office européen des brevets

(11) Publication number: **0 068 693**
**A2**

---

(12)

# EUROPEAN PATENT APPLICATION

(21) Application number: 82303040.8

(22) Date of filing: 11.06.82

(51) Int. Cl.³: **C 12 N 15/00,** C 12 P 21/02, C 12 N 1/20, C 07 C 103/52, C 07 H 21/04, A 61 K 39/135 // C12R1/19

(30) Priority: 16.06.81 US 274103
04.05.82 US 374855

(43) Date of publication of application: 05.01.83
Bulletin 83/1

(84) Designated Contracting States: AT BE CH DE FR GB IT LI LU NL SE

(71) Applicant: GENENTECH, INC., 460 Point San Bruno Boulevard, So. San Francisco California 94080 (US)

(72) Inventor: Kleid, Dennis G., 20 Mosswood Drive, Hillsborough California 94010 (US)
Inventor: Yansura, Daniel G., 125 Pioche, San Francisco California 94134 (US)

(74) Representative: Armitage, Ian Michael et al, MEWBURN ELLIS & CO. 2/3 Cursitor Street, London EC4A 1BQ (GB)

(54) Production of foot and mouth disease vaccine from microbially expressed antigens.

(57) Polypeptides which act as antigens of foot and mouth disease virus (FMDV) are produced by microorganisms which have been transformed with plasmids produced using recombinant DNA technology. A suitable plasmid has a gene sequence (produced by chemical synthesis or by reverse transcription) which encodes a polypeptide comprising one or more immunogenic determinants of one or more FMDV proteins, and optionally a fusion protein preferably linked via a cleavage site, the gene sequence being under the control of a promoter.

For example, an E. Coli K12 strain was transformed with plasmid pFM1. This is based on plasmid pBR322, with the DNA sequence of codons 8 to 210 of the VP$_3$ gene of FMDV type A$_{12}$119ab connected to the LE' protein (from pBR322) so as to be in the same reading frame. This assembly is under the control of the E. Coli trp operon. The transformant E. Coli was cultured and caused to express the LE'—VP$_3$ fusion protein. This was isolated and shown by competitive radio immunoassay to have similar antigenic properties to purified natural VP$_3$ protein.

ACTORUM AG

## PRODUCTION OF FOOT AND MOUTH DISEASE VACCINE FROM MICROBIALLY EXPRESSED ANTIGENS

This invention relates to the use of recombinant DNA technology for the microbial production of foot and mouth disease antigens for use in the preparation of vaccines against foot and mouth disease. In one aspect, the present invention relates to the construction of microbial expression vehicles containing DNA sequences coding for foot and mouth disease antigens operably linked to expression effecting promoter systems and to the expression vehicles so constructed. In another aspect, the present invention relates to microorganisms transformed with such expression vehicles, thus directed in their expression of the DNA sequences referred to above. In yet other aspects, this invention relates to the means and methods of producing    end products of such microbial expression and to means and methods of converting such products to entities, such as vaccines, useful against foot and mouth disease.

## Background of the Invention

### A. Foot and Mouth Disease Antigens

Foot and mouth disease (FMD) is a dreadful, highly contagious disease which affects cloven-footed animals, including valuable, domesticated livestock: cattle, swine, sheep and goats. Its symptoms are manifested as lesions, notably in the mucous membranes of the mouth and on the skin around the hooves. The teats, rumen and myocardium are also frequently involved. Afflicted animals are generally debilitated, as a consequence of lameness and of forced restrictions in eating. Although mortalities are generally low, unless the heart muscle becomes involved, large economic losses are incurred from weight loss and irreversible loss of milk production. In addition, the present methods of preventing transmission of the disease when outbreaks occur, resort to wholesale slaughter of the infected and exposed animals. See the general review by Bachrach, H.L., Viruses and Environment, Academic Press, New York, 1978. Thus, the prevention of outbreaks of foot-and-mouth disease would be of great economic value.

The disease is caused by a virus vector which invades and infects host organism cells, disrupting their useful functions. Within the cell, the viral genome replicates and each copy becomes encapsulated by viral coat protein which is produced within the cell by expression of the appropriate gene codon sequences of the viral RNA. Eventually, the individual cells become loaded with the multiplying virus and burst, discharging the virus entities for infection of neighboring cells.

Foot-and-mouth disease virus (FMDV) is an acid sensitive animal picornavirus of the rhinovirus genus. It has four primary capsid proteins: $VP_1$, $VP_2$, $VP_3$ and $VP_4$ which are thought to have molecular weights of about 30, 30, 27 and 8 kd., respectively. It also has a precursor protein, VPo (ca. 41 kd.), of $VP_2$ and $VP_4$. It apparently also has small amounts of a 52 kd. protein as well as tryptic cleavage products $VP_{3a}$ and $VP_{3b}$. See Perspectives in Virology 10, Raven Press, New York, 1978, particularly Chapter 10 by Bachrach et al., which is incorporated herein by this reference.

The capsid proteins $VP_1$, $VP_2$ and $VP_3$ of FMDV Type $A_{12}$ have been purified and used to study immune response by Bachrach et al., The Journal of Immunology 115, 1636 (1975). These workers concluded that purified $VP_3$ has an essential immunogenic determinant. They found that purified trypsin sensitive protein sensitized guinea pigs for later inducement of antiviral activity with an immunogenic dose of trypsinized FMDV. See also U.S. Patent 4140763 which, along with The Journal of Immunology 115,

-4-

1636 (1975) cited above, is hereby incorporated by reference.

Although FMD vaccine produced from killed (or inactivated) virus is available and has been widely used in many parts of the world since it was first developed in the 1940s, it is costly and difficult to produce. Production problems involving proteolytic digestion of the antigenic portions of the virus, contamination of the production media, and low virus yield have plagued producers. There are also problems during the production involving containment of large quantities of highly contagious material. Incomplete inactivation of the virus in the vaccine products has caused numerous disease outbreaks. Over-inactivation causes severe loss of immunogenic potency. The vaccine preparations themselves are unstable and must be kept cold to prevent further loss of potency. These and other problems are especially acute in the large South American ranches where the availability of present vaccine is most critical.

The present invention may provide solutions to all of these problems because the instant process of vaccine production uses no FMD virus and therefore no virus contamination, inactivation or containment problems exist. The microbiological media used to grow the recombinant organisms are much easier to keep free of contaminants and are much cheaper. The product, which consists essentially only of the FMDV antigenic determinants, is more uniform in effectiveness and potency. The antigenic protein does not inactivate at room temperature; therefore, this vaccine should be much more convenient to use in the field.

B. Recombinant DNA Technology

With the advent of recombinant DNA technology, the controlled bacterial production of useful polypeptides has become possible. The workhorse of recombinant DNA technology is the plasmid, an extra-chromosomal loop of double-stranded DNA found in bacteria, oftentimes in multiple copies per bacterial cell. Included in the information encoded in the plasmid DNA is that required to reproduce the plasmid in daughter cells (i.e., a "replicon") and ordinarily, one or more selection characteristics, such as resistance to antibiotics, which permit clones of the host cell containing the plasmid of interest to be recognized and preferentially grown in selective media. The utility of plasmids, which can be recovered and isolated from the host microorganism, lies in the fact that they can be specifically cleaved by one or another restriction endonuclease or "restriction enzyme," each of which recognizes a different site on the plasmidic DNA. Thereafter heterologous genes or gene fragments may be inserted into the plasmid by endwise joining at the cleavage site or at reconstructed ends adjacent to the cleavage site.

As used herein, the term "heterologous" refers to a gene not ordinarily found in, or a polypeptide sequence ordinarily not produced by, the host microorganism whereas the term "homologous" refers to a gene or polypeptide which is produced in the host microorganism, such as E. coli. DNA recombination is performed outside the microorganisms but the resulting "recombinant" plasmid can be introduced into microorganisms by a process known as transformation and large quantities of the heterologous gene-containing recombinant plasmid obtained by growing the transformant. Moreover, where the gene is properly inserted with reference to portions of the plasmid which govern the transcription and translation of the encoded DNA message, the resulting plasmid or "expression vehicle," when incorporated into the host microorganism, directs the production of the polypeptide sequence for which the inserted gene codes, a process referred to as expression.

Expression is initiated in a region known as the promoter which is recognized by and bound by RNA polymerase. In some cases, as in the trp operon discussed infra, promoter regions are overlapped by "operator" regions to form a combined promoter- operator. Operators are DNA sequences which are recognized by so-called repressor proteins which serve to regulate the frequency of transcription initiation at a particular promoter. The polymerase travels along the DNA, transcribing the information contained in the coding strand from its 5' to 3' end into messenger RNA which is in turn translated into a polypeptide having the amino acid sequence for which the DNA codes. Each amino acid is encoded by a unique nucleotide triplet or "codon" within what may for present purposes be referred to as the "structural gene," i.e. that part which encodes the amino acid sequence of the expressed product. After binding to the promoter, the RNA polymerase first transcribes nucleotides encoding a ribosome binding site, then a translation initiation or "start" signal (ordinarily ATG, which in the resulting messenger RNA becomes AUG), then the nucleotide codons within the structural gene itself. So-called stop codons, if present, are transcribed at the end of the structural gene whereafter the polymerase may form an additional sequence of messenger RNA which, because of the presence of the stop signal, will remain untranslated by the ribosomes. Ribosomes bind to the binding site provided on the messenger RNA, in bacteria ordinarily as the mRNA is being formed, and themselves produce the encoded polypeptide, beginning at the translation start signal and ending at the previously mentioned stop signal. The desired product is produced if the sequences encoding the ribosome binding site are positioned properly with respect to the AUG initiator codon and if all remaining codons follow the initiator codon in phase. The resulting product may be obtained by lysing the host cell and recovering the product by appropriate purification from other microorganism protein(s).

C.  Promoter Systems

As examples, the beta lactamase and lactose promoter systems have been commonly used to initiate and sustain the microbial production of heterologous polypeptides. Details relating to the make-up and construction of these promoter systems have been published by Chang et al., Nature 275, 617-24 (1978) and Itakura et al., Science 198, 1056-63 (1977), which are hereby incorporated by reference. More recently, a system based upon tryptophan, the so-called trp promoter system, has been developed. Details relating to the makeup and construction of this system have been published by Goeddel et al. Nucleic Acids Res. 8: 4057-74 (1980), which is hereby incorporated by reference.

The particular trp promoter system has been constructed having a sequence of double-stranded DNA comprising a trp promoter, nucleotides coding for the trp leader ribosome binding site, and in phase from a first 5' to a second 3' end of the coding strand, nucleotides encoding translation initiation for expression of a structural gene that encodes the amino acid sequence of the heterologous polypeptide. The DNA sequence referred to contains neither a trp attenuator region nor nucleotides coding for the trp E ribosome binding site. Instead, the trp leader ribosome binding site is efficiently used to effect expression of the information encoded by an inserted gene. Cells are transformed by addition of the trp promoter-containing and attenuator-lacking plasmids and grown up in the presence of additive tryptophan. The use of tryptophan-rich media provides sufficient tryptophan to essentially completely repress the trp promoter through trp/repressor interactions, so that cell growth can proceed uninhibited by premature expression of large quantities of heterologous polypeptide encoded by an insert otherwise under the control of the trp promoter system. When the recombinant culture has been grown to the levels appropriate for industrial production of the polypeptide, on the other hand, the external source of tryptophan is depleted, leaving the cell to rely only on the tryptophan that it can itself produce. The result is mild tryptophan limitation and, accordingly, the pathway is derepressed and highly efficient expression of the heterologous insert occurs, unhampered by attenuation because the attenuator region has been deleted from the system. In this manner, the cells are never severely deprived of tryptophan and all proteins, whether they contain tryptophan or not, can be produced in substantial yields. This system is described in more detail by Kleid et al., U.S. patent application Serial No.

133, 296, filed March 24, 1980, corresponding to E.P.O. Publ. No. 0036776, which is hereby incorporated by reference.

D.  State of the Art

Kupper et al., Nature 289, 555 (1981) describe the isolation of cDNA fragments derived from the FMDV strain $O_1$ Kaufenbaren. Boothroyd et al., Nature 290, 800 (1981) describe similar experiments with FMDV $A_{10}$. The DNA coding for the $VP_1$ protein (corresponding to $VP_3$ of other workers and herein) apparently is included in these cDNA fragments. The Kupper et al. group succeeded in subcloning some of the FMDV $VP_3$ gene sequences into an expression vector. In these expression studies, they describe the construction of a plasmid containing the PL promotor, and a portion of the MS2 structural gene in phase (at a Bam HI site) with some coding sequences of the FMDV $VP_1$ ($VP_3$) gene. This construction incorporates amino acids 9 of the $VP_1$ ($VP_3$) structural gene to codon 292 (beyond the $VP_3$ gene) at a Hind III site, into the vector pPLc24. The authors claim to have used this plasmid to transform E. coli and obtain expression controlled by the phage λ PL promotor. They report that some of the transformants may synthesize antigenic material and they estimate production of only 1000 equivalents of antigenic material per cell based on radioimmunoassay using whole bacterial extracts as antigens. The data presented suggests that a substance of unknown antigenic potency was produced in some bacteria containing this plasmid. However, there is no information on whether the substance produced was immunogenic, presumably because the material is inactive or the amounts obtained were insufficient to conduct in vivo animal tests.  See also Kurz et al.,
Nucleic Acids Research 9, 1919 (1981).


Brief Summary of the Invention


The present invention is based upon the discovery that
recombinant DNA technology can be used to successfully and
efficiently produce polypeptides associated with the antigenic
determinants of foot and mouth disease.  Such polypeptides are
produced hereby in amounts sufficient to inoculate  animals in
standard, challenge bioassays and prove the consequential
conferment of immunogenicity to foot and mouth disease.  Thus,
the present invention provides FMD vaccines based upon the
obtention, via recombinant DNA technology, of antigenically
active FMDV polypeptides in amounts and purities sufficient to
confer immunogenicity in susceptible animals.


The FMDV polypeptides described herein are microbially
produced, in accordance with the present invention, both as 1)
stable fusion products which are, in one preferred embodiment,
used directly as immunogens or are specifically cleavable to
provide immunogenic polypeptides unaccompanied by polypeptide
associated with either a precursor to the mature FMDV
protein(s) or the protein(s) of the particular
promoter/microbial system employed, or 2) products of direct
expression not requiring subsequent processing to provide the
mature FMDV polypeptide(s).  The present invention is also
directed to the production of polyantigenic FMDV polypeptides
containing the major antigenic determinant(s) of one or several
FMDV types and subtypes, from functional microbial expression
vehicles having tandem gene sequences coding for multiple
copies of one or more of such polypeptides in specifically
cleavable form.  In any event, the expression products of the present invention containing an immunogenically active constituent of FMDV are microbially produced, recovered and purified to levels required for use in preparing vaccines against FMD. Because the proteins hereof are microbially produced, they are free of extraneous viral proteins which accompany product purified from natural sources. However, the microbial organism employed produces its own proteins which may accompany the desired protein and, after purification, be present in the product in amounts upwards of about five percent by weight. It has been found that these levels do not affect the immunogenicity of the final product and are tolerable in the finished vaccine. Because the microbial proteins are not considered to affect vaccine utility, the final product is undefined in terms of structure and total amount of the microbial proteins. In addition, while the natural FMDV proteins are phosphoproteins (cf. LaTorre et al., Proc. Natl. Acad. Sci. (USA) 77, 7444 (1980)), the microbially produced protein counterparts are not phosphoproteins because the microorganisms lack the enzymes required for protein phosphorylation.

The present invention comprises the FMDV polypeptide(s) so produced and the methods and means of producing them. Specifically, the present invention is directed to FMDV polypeptide(s) comprising the amino acid sequence of at least one immunogenic determinant of mature FMDV protein(s), microbially produced and in amounts sufficient to confer FMD immunogenicity in susceptible animals. The FMDV polypeptide(s) produced hereby optionally contain(s) a specific cleavage site and optionally linked thereto an additional polypeptide sequence derived from the microbially operable promoter system employed or from synthetic DNA. The present invention is further directed to synthetically derived (organic synthesis and/or reverse transcription) gene sequences coding for the polypeptide(s) described above and to replicable microbial expression vehicles harboring said gene sequences in expressible form so that said vehicles are capable of expressing said polypeptide(s). Further, the present invention is directed to microorganisms transformed with the expression vehicles described above and to microbial cultures of such transformed microorganisms, capable of producing the polypeptide(s) described above. In still further aspects, the present invention is directed to various processes useful in preparing said polypeptide(s), gene sequences, expression vehicles, microorganisms and cultures and to specific embodiments thereof. Still further, this invention is directed to the preparation of vaccines useful to confer immunogenicity to FMD in susceptible animals, which incorporate the polypeptide(s) described above, and to the vaccines thus produced, and to the method of using them to inoculate and confer immunogenicity to FMD in susceptible animals.

The term "mature FMDV protein", as used herein, means all antigenically variable strains, types and subtypes of FMD, regardless of origin, unaccompanied by presequence protein or other leader protein not associated with or critical to the antigenically operable character of the viral protein. Such protein contains at least one antigenic determinant of FMDV.

Brief Description of Drawings

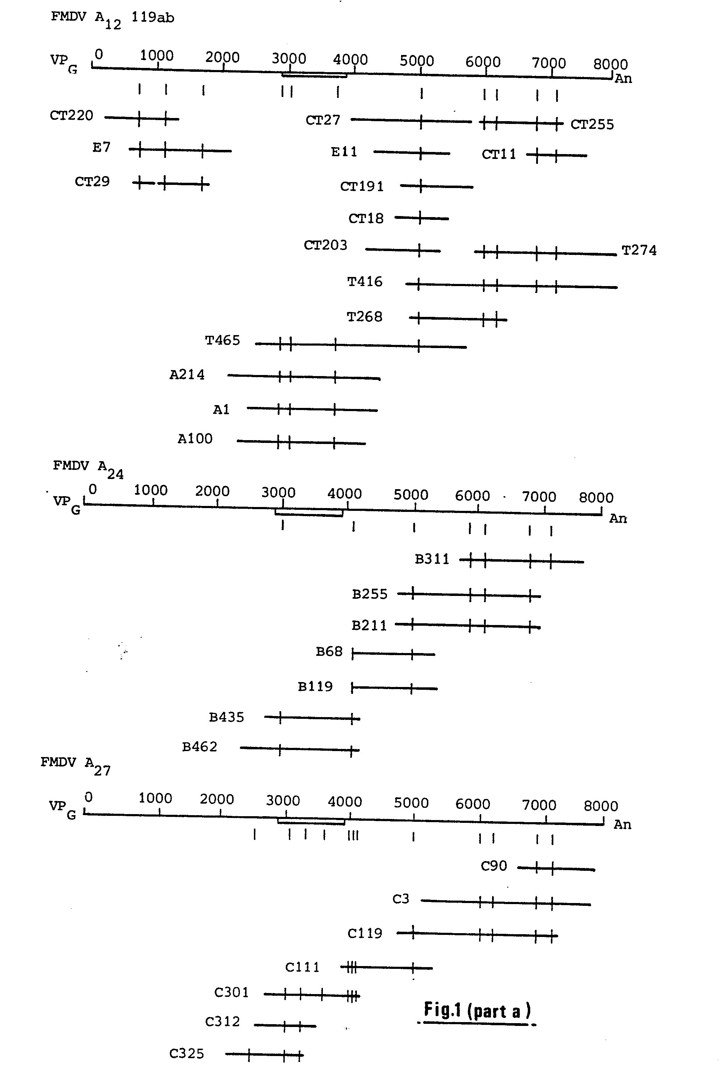

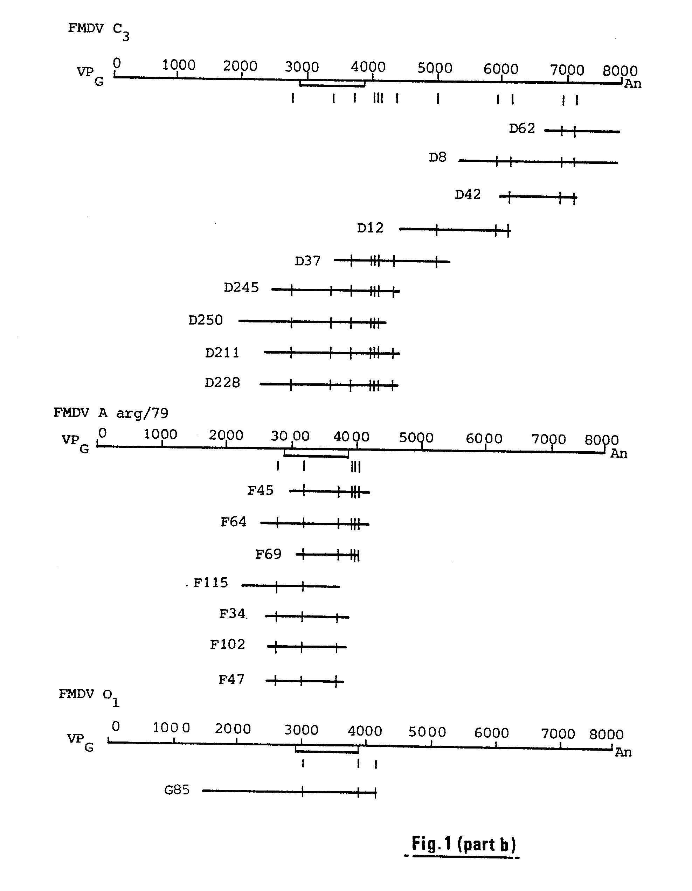

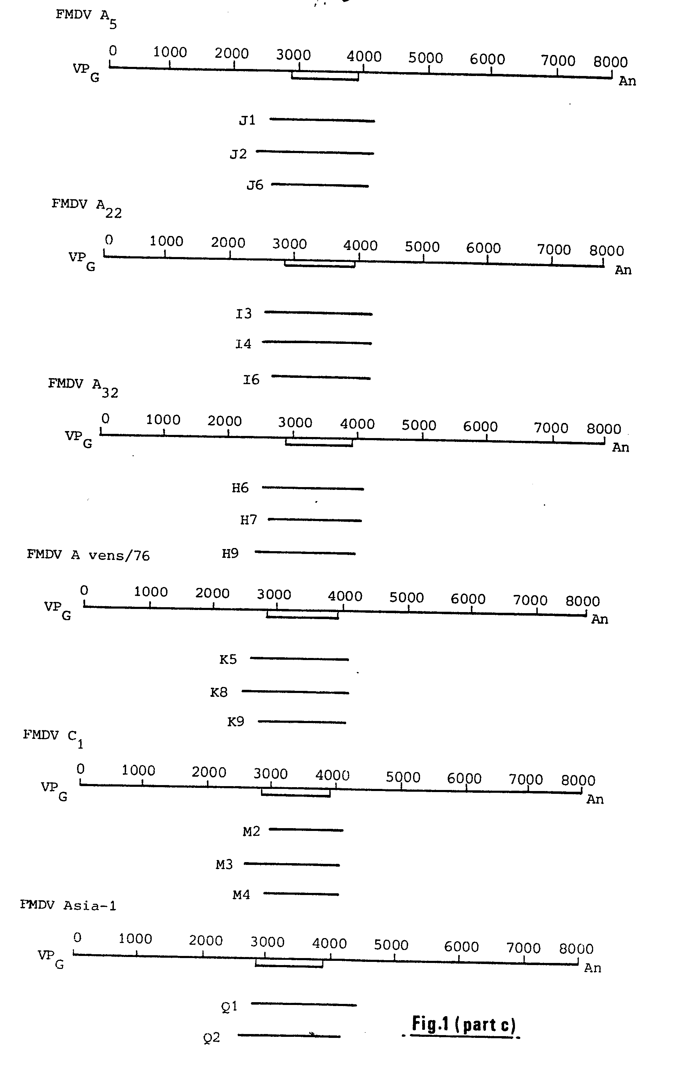

Figure 1 parts a, b and c depicts a series of overlapping cloned cDNA molecules produced using each of twelve different Foot and Mouth Disease Virion RNA molecules as templates for reverse transcriptase. Each of tne cDNA molecules has been given a different designation referring to a particular fully characterized plasmid containing a cDNA insert.

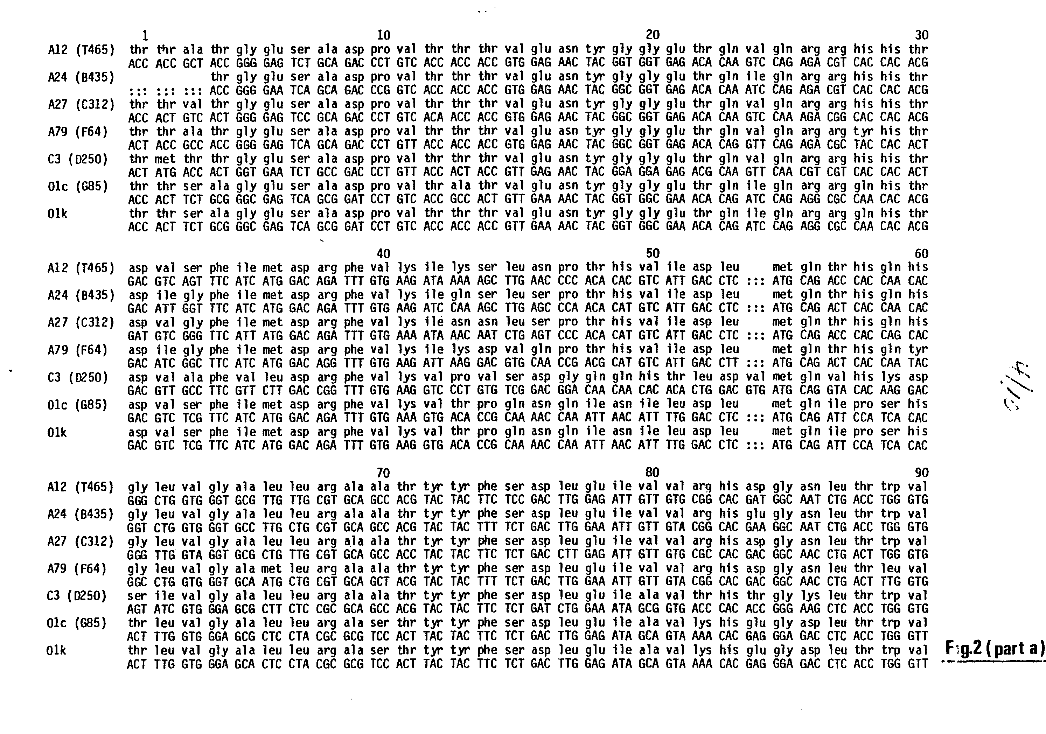

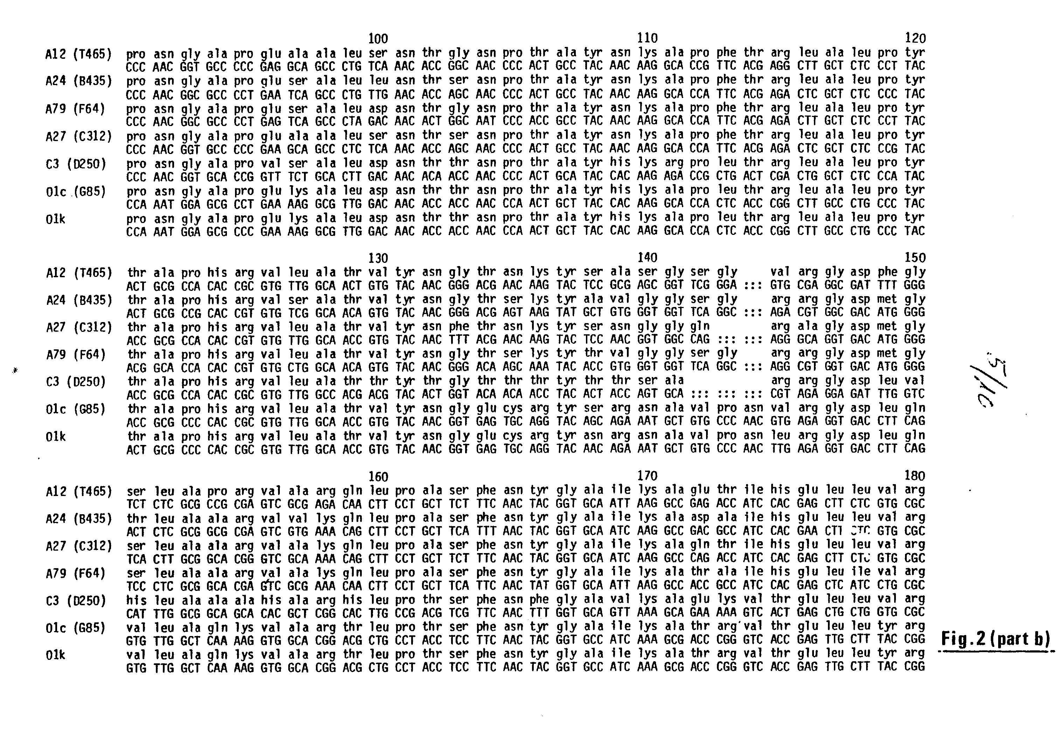

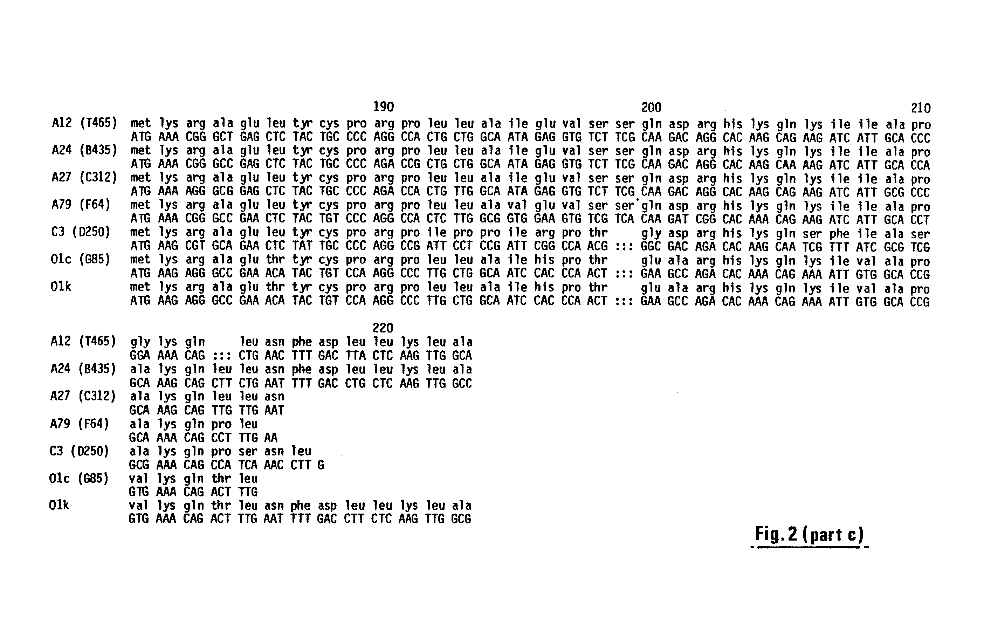

Figure 2 is a compilation of nucleotide and amino acid sequences for the $VP_3$ genes from the various FMDV types and subtypes. They are aligned to show the maximum homology between the types. This is accomplished by adding ::: where one of the types is missing a codon compared to the other types. (The first three codons of $A_{24}$ have not been determined).

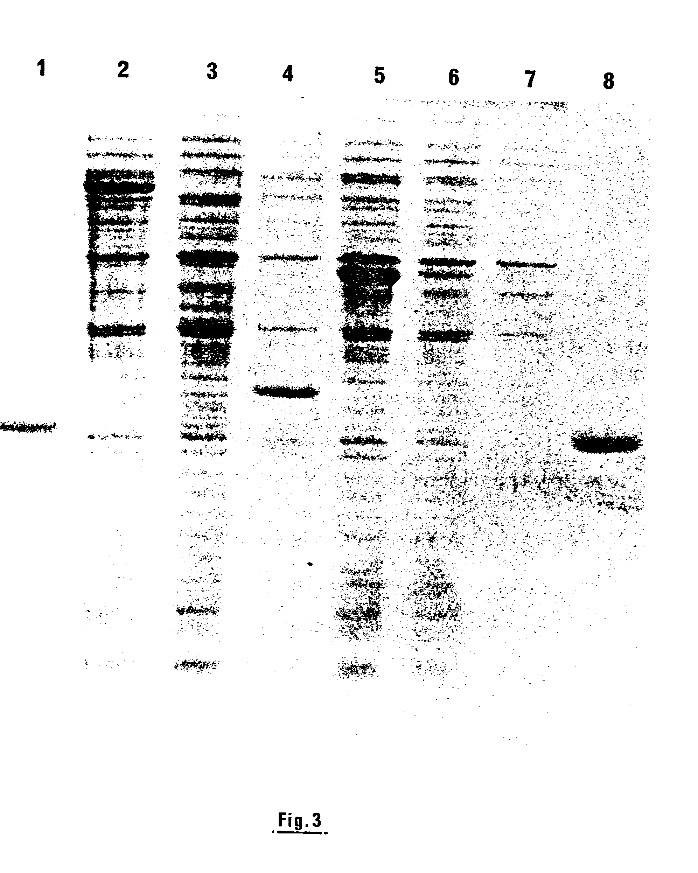

Figure 3 shows an SDS P.A.G.E. of the total protein from each of the vectors: $pFM_1$, $pFM_2$, $pFM_3$, $pFM_{10}$ and $pFM_{20}$, described in detail infra.

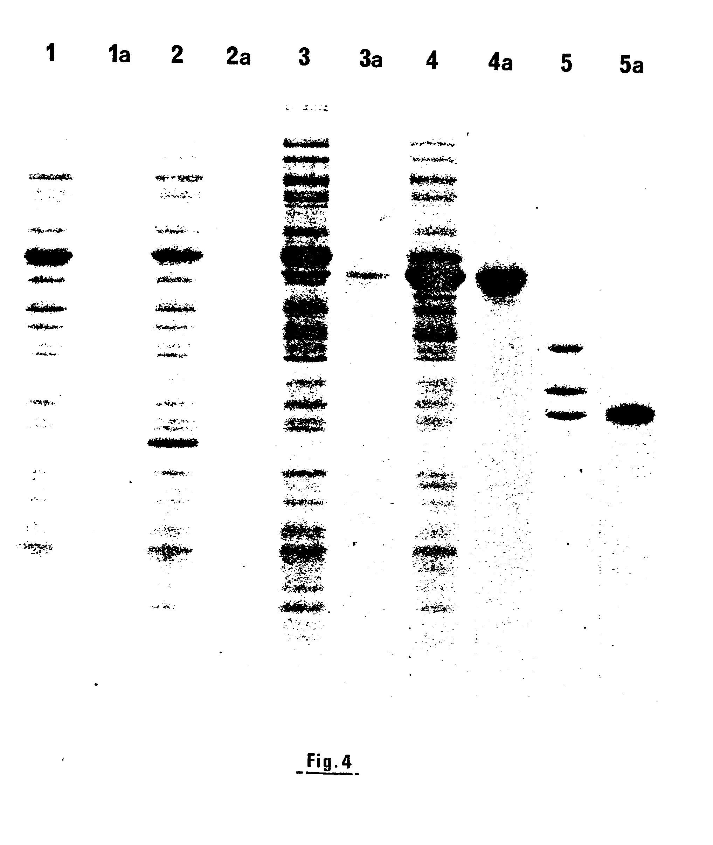

Figure 4 shows an SDS P.A.G.E. analysis of the $LE'-VP_3$ fusion protein. Each sample is shown in pairs, the first lane of the pair shows the total bacterial protein stained with Coomassie blue and the second lane of the pair shows an experiment where the protein was transferred to CNBr treated paper and exposed to $I^{125}$ labelled antibody to $VP_3$.

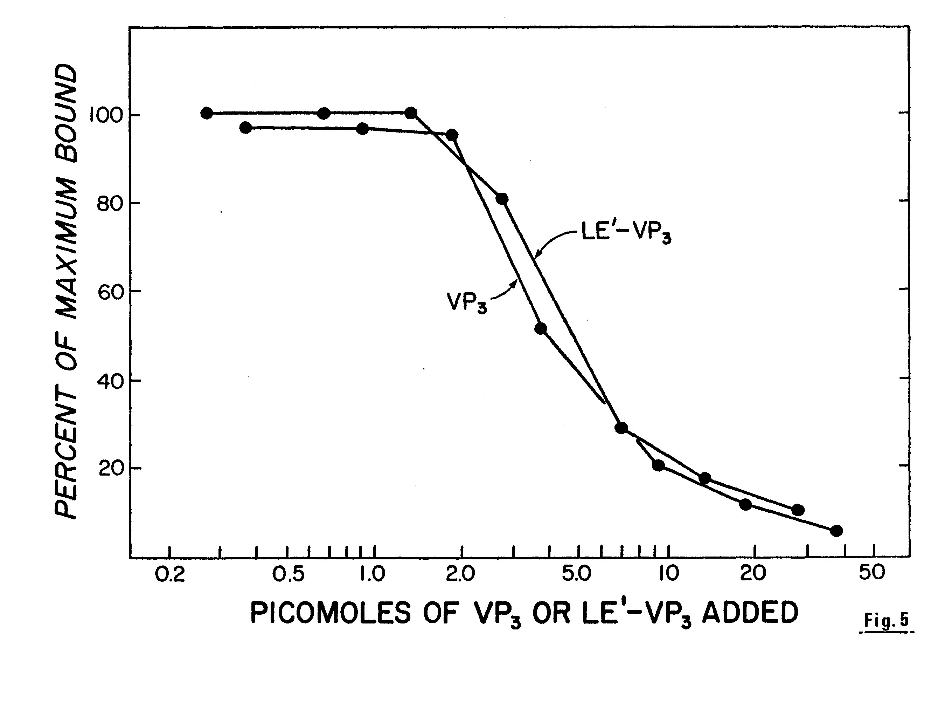

Figure 5 is a comparison of $VP_3$ versus the $pFM_1$ fusion protein's ($LE'VP_3$) ability to inhibit the guinea pig anti-$VP_3$-140S FMDV capsid protein reaction in a competitive radioimmunoassay.

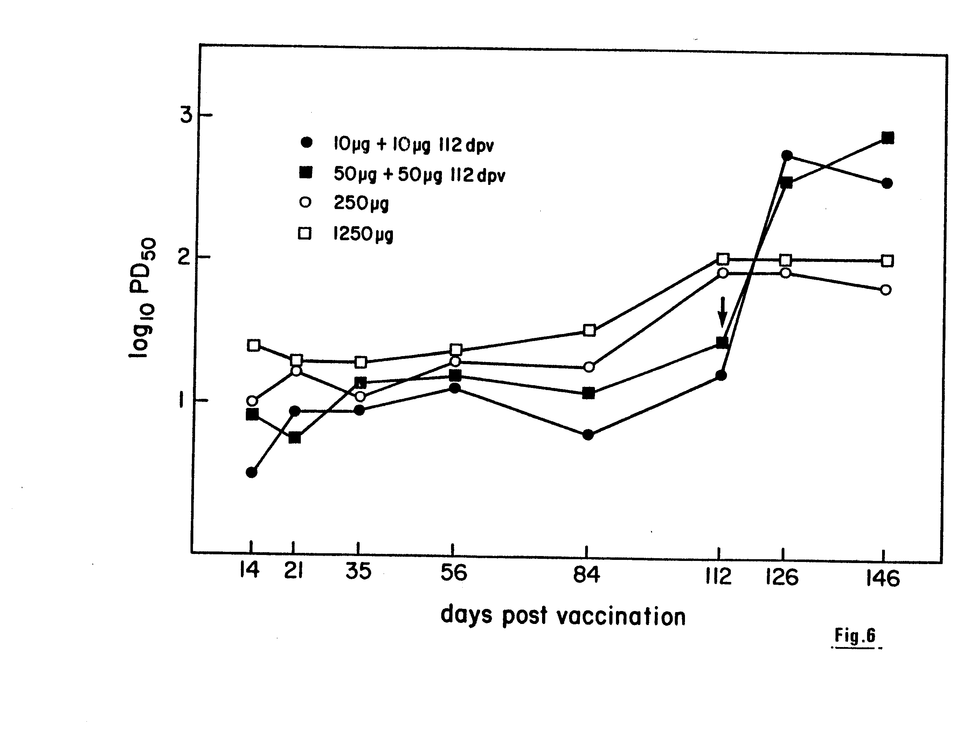

Figure 6 is a comparison of the immunogenic response in cattle to various doses of microbially produced protein. The study shows the level of neutralizing antibody in 36 cattle divided into four groups of nine cattle each. The cattle were vaccinated with the amounts shown in the figure and antibody titers measured over a five month period. The titers are measured by methods described infra.

0068693

## FMD Vaccine

This invention relates to a method whereby antigenic polypeptide(s) of FMDV are produced in useful quantities from microorganisms transformed with specific functional expression vehicles. The utility of this invention is manifest in the production of these polypeptides, the use of them to prepare FMD vaccines, and the subsequent application of these polypeptides, in the form of vaccines, for the prevention of FMD in susceptible animals. The embodiments of this invention demonstrate that these polypeptide(s) possess the desired antigenic properties. These polypeptides are isolated from the microorganisms in which they are produced and purified, optionally with prior cleavage, such as with cyanogen bromide, if fusion polypeptide(s) are produced.

The vaccines of the present invention, incorporating (a) FMDV polypeptide(s) produced as herein described, can be prepared according to known methods, wherein said polypeptide(s) is (are) combined in admixture with a suitable vehicle. Suitable vehicles include, for example, saline solutions, various known adjuvants, or other additives recognized in the art for use in compositions applied to prevent viral infections. Such vaccines will contain an effective amount of the polypeptide(s) hereof and a suitable amount of vehicle in order to prepare a vaccine useful for effective administration to the host. Attention is also directed to New Trends and Developments in Vaccines, Editors: A. Voller and H. Friedman, University Park Press, Baltimore, 1978, which is hereby incorporated by reference, for further background details on the preparation of vaccines.

Because of the unique methods by which the active component of these vaccines are produced, vaccines hereof are likely to be substantially free of extraneous FMD protein(s) and of other viral and cellular components. Therefore, they are less likely to produce the complications of whole killed or attenuated virus vaccine preparations.

The invention, in its preferred embodiments, is described with reference to E. coli, including not only E. coli K-12 strain 294, defined above, but also other known E. coli strains such as E. coli x 1776 and E. coli B, or other microbial strains, many of which are deposited and (potentially) available from recognized microorganism depository institutions, such as the American Type Culture Collection (ATCC)--cf. the ATCC catalogue listing. See also German Offenlegungsschrift 2644432. These other microorganisms include, for example, Bacilli such as Bacillus subtilis and other enterobacteriaceae among which may be mentioned as examples Salmonella typhimurium and Serratia marcesans, utilizing plasmids that can replicate and express heterologous gene sequences in these organisms. Yeast, such as Saccharomyces cerevisiae, may also be employed to advantage as host organism in expressing the proteins, under the control of a yeast promoter. See the copending U.S. patent application by Ronald Hitzeman et al., U.S. Serial No. 237913, filed February 25, 1981, or the corresponding European Patent Application 82300949.3, which are incorporated herein by reference. Thus, the invention is not to be limited to the preferred embodiments described.

Detailed Description

A.  Synthesis of double stranded cDNA

The genome of the Foot and Mouth disease virus (FMDV) consists of a single stranded RNA molecule of approximately 8,000 nucleotides. The molecule is polyadenylated at its 3' end, has a polycytidine sequence near the 5' end, and a viral protein ($VP_G$) covalently attached to the 5' end. The following shows a diagram of the FMDV genome and indicated are the approximate locations of the genes coding for the capsid proteins $VP_1$, $VP_2$, $VP_3$ and $VP_4$.

$$VP_G \underline{\quad CCC \quad} \underline{\quad} \underline{VP_4} \; \underline{VP_2} \; \underline{VP_1} \; \underline{VP_3} \underline{\qquad\qquad\qquad\qquad} A_n$$

These are the proteins that make up the viral coat and are responsible for eliciting an immunogenic response in the infected animal. In order to isolate capsid protein genes it was first necessary to convert the RNA into several overlapping subgenomic double stranded DNA fragments. Using the method of M.J. Grubman, B. Baxt and H.L. Bachrach

[Virology 97, 22-31 (1979)] FMDV RNA was isolated from fourteen different viral strains [FMDV $A_{12}$ 119 ab/PIADC; FMDV $A_5$ Westerwald/58; FMDV $A_{24}$ Cruzeiro Brazil/55; FMDV $A_{27}$ Cundinamarca Columbia/76; FMDV $A_{32}$ Venezuela/70; FMDV A Argentina/79; FMDV A Venceslau Brazil/76; FMDV $A_{22}$/550 Azerbadjan/64 IRAN; FMDV $C_1$ Oberbayern; FMDV $C_3$ Resende Brazil/55; FMDV $O_1$ Campos Brazil/58; FMDV Asia 1 - PAK/57; FMDV SAT 3 Bechouanaland 1/65; FMDV SAT 2 Botswana/77.] In order to synthesize cDNA, reverse transcriptase was primed by a primer-virion RNA complex. The RNA was annealed to one of several oligodeoxynucleotide primers. These primers were of several types: (1) an oligothymidine primer 12-15 nucleotides in length (T primer), (2) a set of synthetic deoxyoligonucleotides eleven bases in length (E primer) based on amino acid sequence data from the $VP_3$ capsid protein (ACTTCTTCTTC, ACTTCCTCTTC, ACTTCTTCCTC, ACTTCCTCCTC, ACCTCTTCTTC, ACCTCCTCTTC, ACCTCTTCCTC, and ACCTCCTCCTC), the synthetic methods used being those of Crea et al. [Proc. Natl. Acad. Sci. (U.S.A.) 75, 5765 (1978)] (3) a set of synthetic deoxyoligonucleotides eight bases in length (8 mer primer) based on amino acid sequence data from the $VP_3$ protein (ATGGTTCC, ATGGTCCC, ATGGTACC, ATGGTACC, GAGAACTA, GAGAATTA, GAAAACTA, and GAAAATTA), (4) nuclease digested calf thymus DNA (CT primer), a complex mixture of oligonucleotides 8-20 bases in length, (5) a synthetic deoxyoligonucleotide (GGCCAGATTT, S primer) complementary to a site near the center of the FMDV $A_{12}$119ab genome (the nucleotide sequence at this site was determined from a cloned FMDV $A_{12}$119ab cDNA segment CT27 described below), and (6) a set of synthetic deoxyoligonucleotides complementary to sites approximately

-18-

200 nucleotides 3' to the $VP_3$ protein gene (V primer,
ACAGCGGCCA, ATGATGGCCA, AAGGACTGGG). The nucleotide
sequence at these sites was determined from a cloned FMDV
$A_{12}$ 119 ab cDNA segment, T465, obtained as described
below.

The primer-RNA annealing and double stranded cDNA
synthesis was carried out as follows. Ethanol precipitated
FMDV RNA 5-10 µg was combined with 1-5 µg primer in 6 µl
$H_2O$, 5 µl of 0.1 M Tris pH 8, 5 µl 10 mM EDTA and 29 µl
$H_2O$ final volume of 45 µl. This was heated to 95° for 5
min, then frozen on dry ice. Added to this was 0.1 mCi
$[\alpha-^{32}P]$dATP dissolved in 50 µl of a solution containing
100 mM Tris HCl pH 8, 10 mM $MgCl_2$, 10 mM
β-mercaptoethanol, 0.3 M NaCl, 2 mM dATP, 2 mM dCTP, 2 mM
dGTP, and 2 mM dTTP. As the solution was melting, 6 µl of
reverse transcriptase (90 units) was added. After 1 hr
incubation at 42°C the reaction was heated to 95°C for 5
min then cooled to 0° and a 100 µl solution containing 50
mM $KH_2PO_4$ pH 7.4, 7 mM $MgCl_2$, 2 mM β-mercaptoethanol
was added followed by 20 µl DNA polymerase I, Klenow
fragment (10 units). After 2-12 hrs at 0°C the mixture was
warmed to 37°C and after 2 hr extracted with 1 volume of
phenol, followed by a chloroform extraction, then 2.5
volumes 95 percent ethanol added. The precipitated sample
was taken up in 200 µl containing 30 mM NaOAc pH 4.6, 50 mM
NaCl, 1 mM $ZnCl_2$, 5 percent glycerol, and 1 µl $S_1$
nuclease (1000 units). After 2 hr at 37°C, the material
was phenol extracted, chloroform extracted and ethanol
precipitated. The sample was taken up in $H_2O$ and
electrophoresed on a 5 percent polyacrylamide gel. The
double stranded cDNA in the size range 1000-5000 base pairs in length was excised from the gel and recovered by electroelution. Based on $^{32}P$ incorporated, the amount of cDNA recovered was calculated and ranged in amounts from 10-300 ng depending on the primer and the template RNA used. The T primer gave consistently higher amounts of cDNA; Table 1 shows the results of a number of experiments.

Table 1

Amounts of ds cDNA synthesized using various primers and vRNA templates.

| FMDV vRNA | | Primer | | Yield of ds-cDNA 1000-5000 bp |
|---|---|---|---|---|
| Type | µg | Type | µg | ng |
| $A_{24}$ | 7 µg | T | 1.5 µg | 200 ng |
| $A_{27}$ | 9 µg | T | 1.5 µg | 90 ng |
| $C_3$ | 9 µg | T | 1.5 µg | 90 ng |
| $O_1$ | 10 µg | T | 1.5 µg | 150 ng |
| $A_{12}$ | 10 µg | T | 1.5 µg | 280 ng |
| $A_{12}$ | 5 µg | E | 1.0 µg | 30 ng |
| $A_{12}$ | 10 µg | CT | 2.0 µg | 7 ng |
| $A_{12}$ | 10 µg | S | 2.0 µg | 60 ng |
| $A_{24}$ | 7 µg | S | 2.0 µg | 30 ng |
| $A_{27}$ | 7 µg | S | 2.0 µg | 5 ng |
| $C_3$ | 5 µg | S | 2.0 µg | 4 ng |
| $A_{12}$ | ~10 µg | V | 5.0 µg | *NC |
| A arg/79 | ~10 µg | V | 5.0 µg | 86 ng |
| $O_1$ | ~10 µg | V | 5.0 µg | 24 ng |
| $A_{24}$ | ~10 µg | V | 5.0 µg | 69 ng |
| $C_3$ | ~10 µg | V | 5.0 µg | 42 ng |
| $A_{27}$ | ~10 µg | V | 5.0 µg | 50 ng |
| $A_{22}$ | ~10 µg | V | 5.0 µg | 28 ng |
| $A_5$ | ~10 µg | V | 5.0 µg | 12 ng |
| $A_{32}$ | ~10 µg | V | 5.0 µg | 36 ng |
| A Venc/76 | ~10 µg | V | 5.0 µg | 23 ng |
| $C_1$ | ~10 µg | V | 5.0 µg | NC |
| Asia-1 | ~10 µg | V | 5.0 µg | NC |
| SAT 2 | ~10 µg | V | 5.0 µg | NC |
| SAT 3 | ~10 µg | V | 5.0 µg | NC |

*NC: not calculated.

-20-

B.  Isolation of plasmids containing FMDV cDNA inserts.

The ds cDNA was attached to the plasmid pBR322 and cloned
in E. coli K12 294 (ATCC Accession No. 31446).  The methods
to accomplish this require tailing the ds cDNA molecule
with a polydeoxycytidine sequence, annealing to the pBR322
previously cut with Pst I and tailed with
polydeoxyguanidine, transformation of E. coli with the DNA
mixture, isolating E. coli colonies containing plasmids
with inserts, and characterizing those inserts by
restriction mapping and DNA sequencing.  The general
methods used to accomplish this have been published, for
example M.P. Wickens et al., J. Biol. Chem. 253, 2483-2495
(1978), D.V. Goeddel et al., Nature 281, 544-548 (1979).
Thirty ng cDNA was dissolved in 40 μl $H_2O$ and combined
with 5 μl 1 M sodium cacodylate buffer pH 7.2, 1 μl of 0.05
M $CoCl_2$, 5 μl of 10 mM dCTP and then treated with 0.5 μl
terminal deoxynucleotidyl transferase (15 units) for 5 min
at 37°C.  The solution was phenol extracted, chloroform
extracted, ethanol precipitated and combined with 300 ng
pBR322 previously cut with Pst I and tailed with dGTP.
This was diluted into 100 μl of annealing solution
containing 0.1 M NaCl, 10 mM Tris HCl pH 7.6, 0.25 mM EDTA
heated to 70°C, then allowed to cool slowly in a water bath
to 37°C overnight.  After 1 hr at room temperature 50 μl of
this solution was used to transform competent E. coli K12
strain 294 by standard procedures.  The bacteria were
spread on tetracycline containing agar plates then colonies
screened for ampicillin resistance.  The colonies that were
$tc^R$ $ap^S$ (95 percent) were grown in 3 ml culture and
plasmids isolated and inserts characterized by restriction
enzyme mapping.  A summary of the data generated by a
series of overlapping cDNA inserts is shown in Figure 1.

In some experiments, a $^{32}P$-labelled DNA fragment coding for the amino terminal part of the $VP_3$ gene, derived from the plasmid FMDV $A_{12}$ T465, was used in a filter hybridization assay. Samples of the plasmid preparations were applied to nitrocellulose filters and hybridized to the labelled fragment according to Grunstein and Hogness (Proc. Natl. Acad. Sci. USA 72, 3961-3965 (1975)). Plasmids containing $VP_3$ gene sequences from the various FMDV strains were identified using this assay. The $VP_3$ genes were further characterized by DNA sequencing. A compilation of the $VP_3$ gene sequences for types $A_{12}$, $A_{24}$, $A_{27}$, A arg/79, $C_3$, O1C and O1K is shown in Figure 2, parts a and b.

C. Expression of antigenic proteins of the FMD virus.

As shown in Figure 1, several of the cDNA inserts contained the FMDV genomic sequences of the immunogenic $VP_3$ protein [H.L. Bachrach et al., J. Immun. 115, 1635-1641 (1975)]. DNA molecules have been cleaved from these inserts using restriction endonucleases and those sequences coding for the immunogenic protein attached to plasmid vectors designed to transcribe the DNA molecules and translate the RNA into protein. Each of these plasmid vectors, or expression vectors, is designed to produce the antigenic protein product in a different form: (1) the antigenic protein expressed as a fusion protein, (2) the antigenic protein expressed directly, (3) the antigenic protein expressed as a polyantigen containing several FMDV antigenic sites expressed as a continuous polyprotein. Other plasmids were designed to express the antigenic $VP_3$ protein of other FMDV types and subtypes: (4) the

antigenic $VP_3$ protein from FMDV $A_{24}$ Cruzeiro Brazil/55 expressed as a fusion protein, (5) the antigenic $VP_3$ protein from FMDV $A_{27}$ Cundinamarca Columbia/76 expressed as a fusion protein, (6) the antigenic $VP_3$ protein from FMDV A Argentina/79 expressed as a fusion protein, (7) the antigenic $VP_3$ protein from FMDV $C_3$ Resende Brazil/55 expressed as a fusion protein, (8) the antigenic $VP_3$ protein from $O_1$ Campos Brazil/58 expressed as a fusion protein.

(1)  The antigenic protein expressed as a fusion protein. The plasmids (or expression vectors) used to express fusion proteins from the FMDV genes were similar to those utilized previously for the production of somatostatin and the human insulin A and B chains (see Kleid et al., U.S.S.N. 133,296, filed March 24, 1980, or the corresponding European Appli-Application 81301227.5 published under Serial No. 0036776, which are hereby incorporated by reference).  In these examples and in the present case the tryptophan operator-promoter is used to express a fusion protein derived from a portion of the tryptophan operon; specifically the first 9 amino acids of the trp leader peptide and the last one third of the trp E protein (the LE' gene).  This gene sequence is ultimately derived from the E. coli tryptophan operon containing the attenuator deletion trp ΔLE 1413 (G.F. Miozzari et al., J. Bacteriology, 1978, 1457-1466).  The construction of these expression vectors is described in detail in Kleid et al., Supra.  Attached to this gene (e.g. LE'), and in the same reading frame, is the gene for the antigenic sites of the FMDV (e.g. $VP_3$).  In order to obtain joining of these two genes in the correct reading frame, a linker DNA molecule has been constructed.

The expression vehicle can be described as consisting of 5
DNA fragments joined together with $T_4$ DNA ligase using
standard procedures and cloned by transformation of E. coli
K12 strain 294. The five fragments have been joined in the
configuration shown below:

The joined DNA fragments 3 and 4, as well as 5 and 1 are
connected by an EcoRI site, whereas fragments 2 and 3 are
isolated from the trp operon joined. Between fragments 4
and 5 there is a PstI site. The junction between fragments
2 and 1 is a filled in EcoRI site (filled in using DNA
polymerase I under standard conditions) and a PstI site
(treated with DNA polymerase I to create a blunt end)
joined together with DNA ligase. The joined ends give the
sequence CAATTC.

DNA fragment 1. This DNA molecule is 3611 base pairs in
length and contains the first 3611 nucleotides of the
plasmid pBR322. The complete nucleotide sequence of this
molecule has been reported (J.G. Sutcliffe, Cold Spring
Harbor Symp. Quant. Biol. 43, 79 (1979)). This fragment
begins with an EcoRI site, contains the tetracycline
resistance gene of pBR322, the origin of replication and a
portion of the β-lactamase gene, up to the Pst I site.
This cleavage site has been removed by DNA polymerase I
nuclease activity using standard conditions.

DNA fragment 2. Fragment 2 contains the promoter and operator of the E. coli tryptophan operon and ends one nucleotide before the ATG initiation codon of the trp L peptide. The sequence and restriction map of this molecule is shown below.

```
   EcoRI                   Hph     Apyl       HgalHhal  Taql
 1 AATTCATGCTGTGGTGTCATGGTCGGTGATCGCCAGGGTGCCGACGCGCATCTCGACTGC
   GTACGACACCACAGTACCAGCCACTAGCGGTCCCACGGCTGCGCGTAGAGCTGACG
                                Mbol Ecorll          Tacl

      HgiA           Acyl     Fnu4hl       Alul
61 ACGGTGCACCAATGCTTCTGGCGTCAGGCAGCCATCGGAAGCTGTGGTATGGCTGTGCAG
   TGCCACGTGGTTACGAAGACCGCAGTCCGTCGGTAGCCTTCGACACCATACCGACACGTC
                     Hgal  . Bbv

            Hph                          Hhal
121 GTCGTAAATCACCGCATAATTCGTGTCGCTCAAGGCGCACTCCCGTTCTGGATAATGTTT
    CAGCATTTAGTGGCGTATTAAGCACAGCGAGTTCCGCGTGAGGGCAAGACCTATTACAAA

    Hhal                                     AlulHindll
181 TTTGCGCCGACATCATAACGGTTCTGGCAAATATTCTGAAATGAGCTGTTGACAATTAAT
    AAACGCGGCTGTAGTATTGCCAAGACCGTTTATAAGACTTTACTCGACAACTGTTAATTA

    Taql     Hindll   RsaI                        Taql
241 CATCGAACTAGTTAACTAGTACGCAAGTTCACGTAAAAAGGGTATCGACA
    GTAGCTTGATCAATTGATCATGCGTTCAAGTGCATTTTTCCCATAGCTGT
             Hpal
```

DNA fragment 3. Fragment 3 contains the structural gene of the LE' fusion protein and is normally linked to fragment 2. The termination codon of the trp E gene has been replaced with an Eco RI site (see Kleid et al., pat. appl. Supra.). The sequence and restriction map of this molecule is shown below.

```
             RsaI                      Bg1llMbolTaql
 1 ATGAAAGCAATTTTCGTACTGAAAGGTTCACTGGACAGAGATCTCGACAGCCGTATTGAA
   TACTTTCGTTAAAAGCATGACTTTCCAAGTGACCTGTCTCTAGAGCTGTCGGCATAACTT
                                            Xho2

        RsaI Mbol    . Alul                      Mbol
61 CTGGAAATGCGTACCGATCATAAAGAGCTGTCTGAACATCTGATGCTGGTTGATCTCGCC
   GACCTTTACGCATGGCTAGTATTTCTCGACAGACTTGTAGACTACGACCAACTAGAGCGG

      Mbol                       HpallFnu4hl       MbolHph
121 CGTAATGATCTGGCACGCATTTGCACCCCCGGCAGCCGCTACGTCGCCGATCTCACCAAA
    GCATTACTAGACCGTGCGTAAACGTGGGGGCCGTCGGCGATGCAGCGGCTAGAGTGGTTT
                                BbvFnu4hl
```

```
     Hindll                        Mn11    Tacl            Hgal
181  GTTGACCGTTATTCCTATGTGATGCACCTCGTCTCTCGCGTAGTCGGCGAACTGCGTCAC
     CAACTGGCAATAAGGATACACTACGTGGAGCAGAGAGCGCATCAGCCGCTTGACGCAGTG


     Mbol Acyl            Tacl                              Hha
241  GATCTTGACGCCCTGCACGCTTATCGCGCCTGTATGAATATGGGGACGTTAAGCGGTGCG
     CTAGAACTGCGGGACGTGCGAATAGCGCGGACATACTTATACCCCTGCAATTCGCCACGC
           Hgal               Hhal


     1    RsaIHhal                    Mn11            Fnu4hlTaclFnu4
301  CCGAAAGTACGCGCTATGCAGTTAATTGCCGAGGCGGAAGGTCGTCGCCGCGGCAGCTAC
     GGCTTTCATGCGCGATACGTCAATTAACGGCTCCGCCTTCCAGCAGCGGCGCCGTCGATG
            Tacl                                    Sac2Fnu4hlBbv


     AluIFnu4hlTacl     Hph  Hhal     MbolTaql            Mbol
361  GGCGGCGCGGTAGGTTATTTCACCGCGCATGGCGATCTCGACACCTGCATTGTGATCCGC
     CCGCCGCGCCATCCAATAAAGTGGCGCGTACCGCTAGAGCTGTGGACGTAACACTAGGCG
         Hhal                    Tacl


     Haell                                                 Hin
421  TCGGCGCTGGTGGAAAACGGTATCGCCACCGTGCAAGCGGGTGCTGGTGTAGTCCTTGAT
     AGCCGCGACCACCTTTTGCCATAGCGGTGGCACGTTCGCCCACGACCACATCAGGAACTA
     Hhal


     f1                                  Tacl   Rsal  HhalHha
481  TCTGTTCCGCAGTCGGAAGCCGACGAAACCCGTAACAAAGCCCGCGCTGTACTGCGCGCT
     AGACAAGGCGTCAGCCTTCGGCTGCTTTGGGCATTGTTTCGGGCGCGACATGACGCGCGA
                                         Hhal            Tacl


     1        Tacl
541  ATTGCCACCGCGCATCATGCACAGG
     TAACGGTGGCGCGTAGTACGTGTCCTTAA
              Hhal                EcoRI
```

<u>DNA fragment 3a</u>.  This DNA molecule contains the first 15 codons of the LE' fusion protein and is normally linked to fragment 2.  The Bgl II recognition sequence at this site has been modified and converted into an Eco RI site.  This was accomplished by ligation, using $T_4$ polynucleotide ligase, of a synthetic DNA molecule (GATCCAGAATTCTG) to the Bgl II cleavage site followed by treatment with Eco RI. The sequence is shown below:

```
ATG AAA GCA ATT TTC GTA CTG AAA GGT TCA CTG GAC AGA GAT CCA G
TAC TTT CGT TAA AAG CAT GAC TTT CCA AGT GAC CTG TCT CTA GGT CTT AA
```

<u>DNA fragment 4</u>.  This fragment contains 7 codons and is bounded by an Eco RI site and a Pst site.  The purpose of this fragment is to bring the FMDV gene sequences into the same reading frame as the LE' gene sequence, and serve as a linker to join the two genes and their RI and Pst ends together. This particular DNA molecule was cleaved from a plasmid that contained the cloned Hepatitis genome [Valenzuela, P. et al., Nature 280, 815-819 (1979)] and has the following sequence:

```
     AATTC CAC TGC CTT CCA CCA AGC TCT GCA
RI       G GTG ACG GAA GGT GGT TCG AG        Pst
```

DNA fragment 4a. This DNA molecule contains 8 codons and is bounded by an Eco RI site and a Pst site. This is a synthetic DNA molecule produced by ligation of five synthetic oligonucleotides (AATTCATGAC, TACTGCTACTGG, TGAATCTGCA, GATTCACCAGTA, GCAGTAGTCATG), synthesized by the methods of Crea et al. Supra. and cloned by the methods of Goeddel et al., Proc. Natl. Acad. Sci. (USA) 76, 106 (1979). It codes for an initial methionine amino acid followed by 7 codons that code for the first 7 amino acids of the $VP_3$ gene. The synthetic sequence differs from the natural sequence in that we have chosen codons preferred by the E. coli host. The purpose of this fragment is to bring the FMDV gene sequences into the same reading frame as the LE' gene sequence, and to serve as a linker to join the two genes and their Eco RI and Pst ends together. A second purpose of this fragment is to provide the coding sequence of the first 7 amino acids of the $VP_3$ protein. A third purpose of this fragment is to encode a methionine amino acid (cleavable with CNBr) between the LE' coded protein and the $VP_3$ protein. The sequence of both the synthetic fragment and the natural FMDV $A_{12}$ 119ab sequence is shown
below:

```
                met thr thr ala thr gly glu ser
            AATTC ATG ACT ACT GCT ACT GGT GAA TCT GCA
synthetic: RI   G TAC TGA TGA CGA TGA CCA CTT AG         Pst

            GGTCA CAA ACC ACC GCT ACC GGG GAG TCT GCA Pst
natural:    CCAGT GTT TGG TGG CGA TGG CCC CTC AG
```

DNA fragment 5: This molecule contains codons 8-210 of the
$VP_3$ gene of the FMDV type $A_{12}$ 119ab. This fragment was
derived from cDNA insert T465 (Fig. 1) using Pst I and Pvu
II. The sequence and restriction map of this molecule is
shown below:

```
    Pst       HPH                            HPH              ACY1
  1 GACCCTGTCACCACCACCGTGGAGAACTACGGTGGTGAGACACAAGTCCAGAGACG
    ACGTCTGGGACAGTGGTGGTGGCACCTCTTGATGCCACCACTCTGTGTTCAGGTCTCTGC


    HPH        ACY1                         MBO11    HIND111
 61 TCACCACACGGACGTCAGTTTCATCATGGACAGATTTGTGAAGATAAAAAGCTTGAACCC
    AGTGGTGTGCCTGCAGTCAAAGTAGTACCTGTCTAAACACTTCTATTTTTCGAACTTGGG
                                                          ALU1


             MNL1
121 CACACACGTCATTGACCTCATGCAGACCCACCAACACGGGCTGGTGGGTGCGTTGTTGCG
    GTGTGTGCAGTAACTGGAGTACGTCTGGGTGGTTGTGCCCGACCACCCACGCAACAACGC


    BBV     RSAI                             FNU4H1             E
181 TGCAGCCACGTACTACTTCTCCGACTTGGAGATTGTTGTGCGGCACGATGGCAATCTGAC
    ACGTCGGTGCATGATGAAGAGGCTGAACCTCTAACAACACGCCGTGCTACCGTTAGACTG
    FNU4H1                                                     A

    COR11          BGL1  MNL1BBV          HPA11
241 CTGGGTGCCCAACGGTGCCCCCGAGGCAGCCCTGTCAAACACCGGCAACCCCACTGCCTA
    GACCCACGGGTTGCCACGGGGGGCTCCGTCGGGACAGTTTGTGGCCGTTGGGGTGACGGAT
    PY1                 AVA1 FNU4H1


                  MNL1                      HHA1      TAC1
301 CAACAAGGCACCGTTCACGAGGCTTGCTCTCCCTTACACTGCGCCACACCGCGTGTTGGC
    GTTGTTCCGTGGCAAGTGCTCCGAACGAGAGGGAATGTGACGCGGTGTGGCGCACAACCG


         RSAI             RSAI TAC1                   MNL1
361 AACTGTGTACAACGGGACGAACAAGTACTCCGCGAGCGGTTCGGGAGTGCGAGGCGATTT
    TTGACACATGTTGCCCTGCTTGTTCATGAGGCGCTCGCCAAGCCCTCACGCTCCGCTAAA


               TAC1FNU4H1HINF1
421 TGGGTCTCTCGCGCCGCGAGTCGCGAGACAACTTCCTGCTTCTTTCAACTACGGTGCAAT
    ACCCAGAGAGCGCGGCGCTCAGCGCTCTGTTGAAGGACGAAGAAAGTTGATGCCACGTTA
               HHA1TAC1   TAC1
```

```
        HAE111         ALU1    MST1              DDE1HGIA TTHIII1
481 TAAGGCCGAGACCATCCACGAGCTTCTCGTGCGCATGAAACGGGCTGAGCTCTACTGCCC
    ATTCCGGCTCTGGTAGGTGCTCGAAGAGCACGCGTACTTTGCCCGACTCGAGATGACGGG
                                      HHA1         SAC1ALU1      E

    APYHAE111          MNL1 MB011                    MB011
541 CAGGCCACTGCTGGCAATAGAGGTGTCTTCGCAAGACAGGCACAAGCAGAAGATCATTGC
    GTCCGGTGACGACCGTTATCTCCACAGAAGCGTTCTGTCCGTGTTCGTCTTCTAGTAACG
    CORHAE1                                            MB01

        HPA11      PVUII
601 ACCCGGAAAACAG
    TGGGCCTTTTGTC
```

The fusion protein expression vectors constructed are pFM1-3 and are listed in Table 2. Each can be described as consisting of 5 DNA fragments joined together by standard methods (fragments 2 and 3 were isolated joined). The plasmid pFM1 expresses from the trp promoter-operator system a protein consisting of 190 amino acids from the LE' gene construction linked to 6 amino acids from the linker molecule (fragment 4), linked to 205 amino acids from the $VP_3$ FMDV $A_{12}$ (codons 7-211), linked to 4 amino acids from pBR322 for a total of 405 amino acids. Translation stops at a TGA codon, the fifth codon of pBR322 in that reading frame. The plasmid pFM2 expresses a protein consisting of 190 amino acids from the LE' gene, linked to a methionine amino acid (CNBr cleavable), linked to the amino acids 1-211 of the $VP_3$ FMDV $A_{12}$ (codons 1-7 are derived from synthetic DNA, 8-211 are natural codons), linked to 4 amino acids from pBR322 for a total of 406 amino acids. The plasmid pFM3 expresses a protein consisting of 17 amino acids from the LE' gene construction (fragment 3a), linked to a methionine amino acid (CNBr cleavable), linked to the amino acids 1-211 of the $VP_3$ FMDV $A_{12}$, linked to 4 amino acids from pBR322 for a total of 233 amino acids.

Table 2

Fusion Protein Expression Vectors Constructed

| Expression Vector | DNA fragments | MW of fusion protein |
|---|---|---|
| pFM1 | 1, 2, 3, 4, 5 | 44.4 Kd |
| pFM2 | 1, 2, 3, 4a, 5 | 44.4 Kd |
| pFM3 | 1, 2, 3a, 4a, 5 | 25.5 Kd |

The amino acid sequence data was deduced from DNA sequence results and is given below.

Fusion protein expressed from $pFM_1$.

```
1                                         10
met lys ala ile phe val leu lys gly ser leu asp arg asp leu
AUG AAA GCA AUU UUC GUA CUG AAA GGU UCA CUG GAC AGA GAU CUC

          20                                        30
asp ser arg ile glu leu glu met arg thr asp his lys glu leu
GAC AGC CGU AUU GAA CUG GAA AUG CGU ACC GAU CAU AAA GAG CUG

                              40
ser glu his leu met leu val asp leu ala arg asn asp leu ala
UCU GAA CAU CUG AUG CUG GUU GAU CUC GCC CGU AAU GAU CUG GCA

          50                                        60
arg ile cys thr pro gly ser arg tyr val ala asp leu thr lys
CGC AUU UGC ACC CCC GGC AGC CGC UAC GUC GCC GAU CUC ACC AAA

                              70
val asp arg tyr ser tyr val met his leu val ser arg val val
GUU GAC CGU UAU UCC UAU GUG AUG CAC CUC GUC UCU CGC GUA GUC

          80                                        90
gly glu leu arg his asp leu asp ala leu his ala tyr arg ala
GGC GAA CUG CGU CAC GAU CUU GAC GCC CUG CAC GCU UAU CGC GCC

                              100
cys met asn met gly thr leu ser gly ala pro lys val arg ala
UGU AUG AAU AUG GGG ACG UUA AGC GGU GCG CCG AAA GUA CGC GCU

          110                                       120
met gln leu ile ala glu ala glu gly arg arg arg gly ser tyr
AUG CAG UUA AUU GCC GAG GCG GAA GGU CGU CGC CGC GGC AGC UAC

                              130
gly gly ala val gly tyr phe thr ala his gly asp leu asp thr
GGC GGC GCG GUA GGU UAU UUC ACC GCG CAU GGC GAU CUC GAC ACC

          140                                       150
cys ile val ile arg ser ala leu val glu asn gly ile ala thr
UGC AUU GUG AUC CGC UCG GCG CUG GUG GAA AAC GGU AUC GCC ACC

                              160
val gln ala gly ala gly val val leu asp ser val pro gln ser
GUG CAA GCG GGU GCU GGU GUA GUC CUU GAU UCU GUU CCG CAG UCG
```

```
                      170                                        180
glu ala asp glu thr arg asn lys ala arg ala val leu arg ala
GAA GCC GAC GAA ACC CGU AAC AAA GCC CGC GCU GUA CUG CGC GCU


                                                190
ile ala thr ala his his ala gln glu phe his cys leu pro pro
AUU GCC ACC GCG CAU CAU GCA CAG GAA UUC CAC UGC CUU CCA CCA


                      200                                        210
ser ser ala asp pro val thr thr thr val glu asn tyr gly gly
AGC UCU GCA GAC CCU GUC ACC ACC ACC GUG GAG AAC UAC GGU GGU


                                                220
glu thr gln val gln arg arg his his thr asp val ser phe ile
GAG ACA CAA GUC CAG AGA CGU CAC CAC ACG GAC GUC AGU UUC AUC


                      230                                        240
met asp arg phe val lys ile lys ser leu asn pro thr his val
AUG GAC AGA UUU GUG AAG AUA AAA AGC UUG AAC CCC ACA CAC GUC


                                                250
ile asp leu met gln thr his gln his gly leu val gly ala leu
AUU GAC CUC AUG CAG ACC CAC CAA CAC GGG CUG GUG GGU GCG UUG


                      260                                        270
leu arg ala ala thr tyr tyr phe ser asp leu glu ile val val
UUG CGU GCA GCC ACG UAC UAC UUC UCC GAC UUG GAG AUU GUU GUG


                                                280
arg his asp gly asn leu thr trp val pro asn gly ala pro glu
CGG CAC GAU GGC AAU CUG ACC UGG GUG CCC AAC GGU GCC CCC GAG


                      290                                        300
ala ala leu ser asn thr gly asn pro thr ala tyr asn lys ala
GCA GCC CUG UCA AAC ACC GGC AAC CCC ACU GCC UAC AAC AAG GCA


                                                310
pro phe thr arg leu ala leu pro tyr thr ala pro his arg val
CCG UUC ACG AGG CUU GCU CUC CCU UAC ACU GCG CCA CAC CGC GUG


                      320                                        330
leu ala thr val tyr asn gly thr asn lys tyr ser ala ser gly
UUG GCA ACU GUG UAC AAC GGG ACG AAC AAG UAC UCC GCG AGC GGU


                                                340
ser gly val arg gly asp phe gly ser leu ala pro arg val ala
UCG GGA GUG CGA GGC GAU UUU GGG UCU CUC GCG CCG CGA GUC GCG


                      350                                        360
arg gln leu pro ala ser phe asn tyr gly ala ile lys ala glu
AGA CAA CUU CCU GCU UCU UUC AAC UAC GGU GCA AUU AAG GCC GAG


                                                370
thr ile his glu leu leu val arg met lys arg ala glu leu tyr
ACC AUC CAC GAG CUU CUC GUG CGC AUG AAA CGG GCU GAG CUC UAC


                      380                                        390
cys pro arg pro leu leu ala ile glu val ser ser gln asp arg
UGC CCC AGG CCA CUG CUG GCA AUA GAG GUG UCU UCG CAA GAC AGG
```

```
                                        400                    405
his lys gln lys ile ile ala pro gly lys gln asn ser his val
CAC AAG CAG AAG AUC AUU GCA CCC GGA AAA CAG AAU UCU CAU GUU

OP
UGA
```

Fusion protein expressed from pFM₂.

```
  1                                     10
met lys ala ile phe val leu lys gly ser leu asp arg asp leu
AUG AAA GCA AUU UUC GUA CUG AAA GGU UCA CUG GAC AGA GAU CUC

                    20                                     30
asp ser arg ile glu leu glu met arg thr asp his lys glu leu
GAC AGC CGU AUU GAA CUG GAA AUG CGU ACC GAU CAU AAA GAG CUG

                                        40
ser glu his leu met leu val asp leu ala arg asn asp leu ala
UCU GAA CAU CUG AUG CUG GUU GAU CUC GCC CGU AAU GAU CUG GCA

                    50                                     60
arg ile cys thr pro gly ser arg tyr val ala asp leu thr lys
CGC AUU UGC ACC CCC GGC AGC CGC UAC GUC GCC GAU CUC ACC AAA

                                        70
val asp arg tyr ser tyr val met his leu val ser arg val val
GUU GAC CGU UAU UCC UAU GUG AUG CAC CUC GUC UCU CGC GUA GUC

                    80                                     90
gly glu leu arg his asp leu asp ala leu his ala tyr arg ala
GGC GAA CUG CGU CAC GAU CUU GAC GCC CUG CAC GCU UAU CGC GCC

                                        100
cys met asn met gly thr leu ser gly ala pro lys val arg ala
UGU AUG AAU AUG GGG ACG UUA AGC GGU GCG CCG AAA GUA CGC GCU

                    110                                    120
met gln leu ile ala glu ala glu gly arg arg arg gly ser tyr
AUG CAG UUA AUU GCC GAG GCG GAA GGU CGU CGC CGC GGC AGC UAC

                                        130
gly gly ala val gly tyr phe thr ala his gly asp leu asp thr
GGC GGC GCG GUA GGU UAU UUC ACC GCG CAU GGC GAU CUC GAC ACC

                    140                                    150
cys ile val ile arg ser ala leu val glu asn gly ile ala thr
UGC AUU GUG AUC CGC UCG GCG CUG GUG GAA AAC GGU AUC GCC ACC

                                        160
val gln ala gly ala gly val val leu asp ser val pro gln ser
GUG CAA GCG GGU GCU GGU GUA GUC CUU GAU UCU GUU CCG CAG UCG

                    170                                    180
glu ala asp glu thr arg asn lys ala arg ala val leu arg ala
GAA GCC GAC GAA ACC CGU AAC AAA GCC CGC GCU GUA CUG CGC GCU

                                        190
ile ala thr ala his his ala gln glu phe met thr thr ala thr
AUU GCC ACC GCG CAU CAU GCA CAG GAA UUC AUG ACU ACU GCU ACU
```

```
                200                                            210
gly glu ser ala asp pro val thr thr thr val glu asn tyr gly
GGU GAA UCU GCA GAC CCU GUC ACC ACC ACC GUG GAG AAC UAC GGU


                                            220
gly glu thr gln val gln arg arg his his thr asp val ser phe
GGU GAG ACA CAA GUC CAG AGA CGU CAC CAC ACG GAC GUC AGU UUC


                230                                            240
ile met asp arg phe val lys ile lys ser leu asn pro thr his
AUC AUG GAC AGA UUU GUG AAG AUA AAA AGC UUG AAC CCC ACA CAC


                                            250
val ile asp leu met gln thr his gln his gly leu val gly ala
GUC AUU GAC CUC AUG CAG ACC CAC CAA CAC GGG CUG GUG GGU GCG


                260                                            270
leu leu arg ala ala thr tyr tyr phe ser asp leu glu ile val
UUG UUG CGU GCA GCC ACG UAC UAC UUC UCC GAC UUG GAG AUU GUU


                                            280
val arg his asp gly asn leu thr trp val pro asn gly ala pro
GUG CGG CAC GAU GGC AAU CUG ACC UGG GUG CCC AAC GGU GCC CCC


                290                                            300
glu ala ala leu ser asn thr gly asn pro thr ala tyr asn lys
GAG GCA GCC CUG UCA AAC ACC GGC AAC CCC ACU GCC UAC AAC AAG


                                            310
ala pro phe thr arg leu ala leu pro tyr thr ala pro his arg
GCA CCG UUC ACG AGG CUU GCU CUC CCU UAC ACU GCG CCA CAC CGC


                320                                            330
val leu ala thr val tyr asn gly thr asn lys tyr ser ala ser
GUG UUG GCA ACU GUG UAC AAC GGG ACG AAC AAG UAC UCC GCG AGC


                                            340
gly ser gly val arg gly asp phe gly ser leu ala pro arg val
GGU UCG GGA GUG CGA GGC GAU UUU GGG UCU CUC GCG CCG CGA GUC


                350                                            360
ala arg gln leu pro ala ser phe asn tyr gly ala ile lys ala
GCG AGA CAA CUU CCU GCU UCU UUC AAC UAC GGU GCA AUU AAG GCC


                                            370
glu thr ile his glu leu leu val arg met lys arg ala glu leu
GAG ACC AUC CAC GAG CUU CUC GUG CGC AUG AAA CGG GCU GAG CUC


                380                                            390
tyr cys pro arg pro leu leu ala ile glu val ser ser gln asp
UAC UGC CCC AGG CCA CUG CUG GCA AUA GAG GUG UCU UCG CAA GAC


                                            400
arg his lys gln lys ile ile ala pro gly lys gln asn ser his
AGG CAC AAG CAG AAG AUC AUU GCA CCC GGA AAA CAG AAU UCU CAU


406
val OP
GUU UGA
```

Fusion protein expressed from pFM3.

```
 1                                    10
met lys ala ile phe val leu lys gly ser leu asp arg asp pro
AUG AAA GCA AUU UUC GUA CUG AAA GGU UCA CUG GAC AGA GAU CCA

                    20                                    30
glu phe met thr thr ala thr gly glu ser ala asp pro val thr
GAA UUC AUG ACU ACU GCU ACU GGU GAA UCU GCA GAC CCU GUC ACC

                              40
thr thr val glu asn tyr gly gly glu thr gln val gln arg arg
ACC ACC GUG GAG AAC UAC GGU GGU GAG ACA CAA GUC CAG AGA CGU

                    50                                    60
his his thr asp val ser phe ile met asp arg phe val lys ile
CAC CAC ACG GAC GUC AGU UUC AUC AUG GAC AGA UUU GUG AAG AUA

                                    70
lys ser leu asn pro thr his val ile asp leu met gln thr his
AAA AGC UUG AAC CCC ACA CAC GUC AUU GAC CUC AUG CAG ACC CAC

                    80                                    90
gln his gly leu val gly ala leu leu arg ala ala thr tyr tyr
CAA CAC GGG CUG GUG GGU GCG UUG UUG CGU GCA GCC ACG UAC UAC

                                    100
phe ser asp leu glu ile val val arg his asp gly asn leu thr
UUC UCC GAC UUG GAG AUU GUU GUG CGG CAC GAU GGC AAU CUG ACC

                    110                                    120
trp val pro asn gly ala pro glu ala ala leu ser asn thr gly
UGG GUG CCC AAC GGU GCC CCC GAG GCA GCC CUG UCA AAC ACC GGC

                                    130
asn pro thr ala tyr asn lys ala pro phe thr arg leu ala leu
AAC CCC ACU GCC UAC AAC AAG GCA CCG UUC ACG AGG CUU GCU CUC

                    140                                    150
pro tyr thr ala pro his arg val leu ala thr val tyr asn gly
CCU UAC ACU GCG CCA CAC CGC GUG UUG GCA ACU GUG UAC AAC GGG

                                    160
thr asn lys tyr ser ala ser gly ser gly val arg gly asp phe
ACG AAC AAG UAC UCC GCG AGC GGU UCG GGA GUG CGA GGC GAU UUU

                    170                                    180
gly ser leu ala pro arg val ala arg gln leu pro ala ser phe
GGG UCU CUC GCG CCG CGA GUC GCG AGA CAA CUU CCU GCU UCU UUC

                                    190
asn tyr gly ala ile lys ala glu thr ile his glu leu leu val
AAC UAC GGU GCA AUU AAG GCC GAG ACC AUC CAC GAG CUU CUC GUG

                    200                                    210
arg met lys arg ala glu leu tyr cys pro arg pro leu leu ala
CGC AUG AAA CGG GCU GAG CUC UAC UGC CCC AGG CCA CUG CUG GCA

                                    220
ile glu val ser ser gln asp arg his lys gln lys ile ile ala
AUA GAG GUG UCU UCG CAA GAC AGG CAC AAG CAG AAG AUC AUU GCA
```

```
                 230         - 233
pro gly lys gln asn ser his val OP
CCC GGA AAA CAG AAU UCU CAU GUU UGA
```

(2)  The antigenic protein expressed directly.

The plasmid  (or expression vehicle ) used to express the VP$_3$ gene directly is similar to that previously used in the production of human growth hormone (Kleid et al., pat. appln. Supra.) and leukocyte interferon (Goeddel et al., Nature 287, 411-416 (1980), which is hereby incorporated by reference).

The expression vehicle can be described as consisting of 4 DNA fragments joined together with T$_4$ DNA ligase using standard procedures and cloned by transformation of E. coli K12 strain 294.  The fragments have been joined in the configuration shown below:

The junctions between fragments 2a and 4a and also between fragments 5 and 1a contain EcoRI sites.  The junction between fragments 4a and 5 contains a Pst I site.  The junction between fragments 1a and 2a contains the sequence GAATTAATTC created during one of the steps of the plasmid construction process by treatment with DNA polymerase and blunt end ligation using standard conditions.

The direct expression plasmid vector constructed contains the previously described DNA fragments 4a and 5 and the entire plasmid, pBR322 (fragment 1a). The fragment 2a contains the tryptophan promoter, operator, mRNA initiation site, ribosome binding site coding sequence and a Xba I site introduced in place of the normal initiation codon.

DNA fragment 2a. Fragment 2a contains the promoter and operator of the E. coli tryptophan operon and ends with an RI-Xba I site before the ATG of the trp L peptide. The sequence and restriction map of this molecule is shown below:

```
    EcoR1                    Hph         Apyl      HgalHhal  Taql
  1 AATTCATGCTGTGGTGTCATGGTCGGTGATCGCCAGGGTGCCGACGCGCATCTCGACTGC
    GTACGACACCACAGTACCAGCCACTAGCGGTCCCACGGCTGCGCGTAGAGCTGACG
                                    Mbol Ecorll        Tacl

       HgiA                  Acyl     Bbv        Alul
 61 ACGGTGCACCAATGCTTCTGGCGTCAGGCAGCCATCGGAAGCTGTGGTATGGCTGTGCAG
    TGCCACGTGGTTACGAAGACCGCAGTCCGTCGGTAGCCTTCGACACCATACCGACACGTC
                           Hgal   Fnu4hl

          Hph                              Hhal
121 GTCGTAAATCACCGCATAATTCGTGTCGCTCAAGGCGCACTCCCGTTCTGGATAATGTTT
    CAGCATTTAGTGGCGTATTAAGCACAGCGAGTTCCGCGTGAGGGCAAGACCTATTACAAA

       Hhal                                        AlulHindll
181 TTTGCGCCGACATCATAACGGTTCTGGCAAATATTCTGAAATGAGCTGTTGACAATTAAT
    AAACGCGGCTGTAGTATTGCCAAGACCGTTTATAAGACTTTACTCGACAACTGTTAATTA

    Taql    Hpal    RsaI.                              Xbal
241 CATCGAACTAGTTAACTAGTACGCAAGTTCACGTAAAAAGGGTATCTAG
    GTAGCTTGATCAATTGATCATGCGTTCAAGTGCATTTTTCCCATAGATCTTAA
              Hindll                                    EcoR1
```

The direct expression vector constructed is pFM 10. It can be described as consisting of 4 DNA fragments joined together by standard methods using $T_4$ DNA ligase and cloned by transformation of E. coli K12 strain 294. It initiates expression of the protein from a methionine and continues with amino acids 1-211 of the $VP_3$ gene linked to 4 amino acids from pBR322 for a total of 216 amino acids.

In order to express the precise VP₃ protein, a synthetic
linker, CTCTAGAG, can be ligated to the Pvu II site of
Fragment 5 above.

Table 2

Direct Expression Vector Constructed.

| Expression Vector | DNA Fragments | MW of protein |
|---|---|---|
| pFM10 | 1a, 2a, 4a, 5 | 23.6 Kd |

The directly expressed VP₃ from pFM₁₀.

```
     1                                    10
met thr thr ala thr gly glu ser ala asp pro val thr thr thr
AUG ACU ACU GCU ACU GGU GAA UCU GCA GAC CCU GUC ACC ACC ACC

             20                                    30
val glu asn tyr gly gly glu thr gln val gln arg arg his his
GUG GAG AAC UAC GGU GGU GAG ACA CAA GUC CAG AGA CGU CAC CAC

                           40
thr asp val ser phe ile met asp arg phe val lys ile lys ser
ACG GAC GUC AGU UUC AUC AUG GAC AGA UUU GUG AAG AUA AAA AGC

             50                                    60
leu asn pro thr his val ile asp leu met gln thr his gln his
UUG AAC CCC ACA CAC GUC AUU GAC CUC AUG CAG ACC CAC CAA CAC

                           70
gly leu val gly ala leu leu arg ala ala thr tyr tyr phe ser
GGG CUG GUG GGU GCG UUG UUG CGU GCA GCC ACG UAC UAC UUC UCC

             80                                    90
asp leu glu ile val val arg his asp gly asn leu thr trp val
GAC UUG GAG AUU GUU GUG CGG CAC GAU GGC AAU CUG ACC UGG GUG

                          100
pro asn gly ala pro glu ala ala leu ser asn thr gly asn pro
CCC AAC GGU GCC CCC GAG GCA GCC CUG UCA AAC ACC GGC AAC CCC

             110                                   120
thr ala tyr asn lys ala pro phe thr arg leu ala leu pro tyr
ACU GCC UAC AAC AAG GCA CCG UUC ACG AGG CUU GCU CUC CCU UAC

                          130
thr ala pro his arg val leu ala thr val tyr asn gly thr asn
ACU GCG CCA CAC CGC GUG UUG GCA ACU GUG UAC AAC GGG ACG AAC

             140                                   150
lys tyr ser ala ser gly ser gly val arg gly asp phe gly ser
AAG UAC UCC GCG AGC GGU UCG GGA GUG CGA GGC GAU UUU GGG UCU
```

```
                                  160
leu ala pro arg val ala arg gln leu pro ala ser phe asn tyr
CUC GCG CCG CGA GUC GCG AGA CAA CUU CCU GCU UCU UUC AAC UAC

                170                                    180
gly ala ile lys ala glu thr ile his glu leu leu val arg met
GGU GCA AUU AAG GCC GAG ACC AUC CAC GAG CUU CUC GUG CGC AUG

                                  190
lys arg ala glu leu tyr cys pro arg pro leu leu ala ile glu
AAA CGG GCU GAG CUC UAC UGC CCC AGG CCA CUG CUG GCA AUA GAG

                200                                    210
val ser ser gln asp arg his lys gln lys ile ile ala pro gly
GUG UCU UCG CAA GAC AGG CAC AAG CAG AAG AUC AUU GCA CCC GGA

                216
lys gln asn ser his val OP
AAA CAG AAU UCU CAU GUU UGA
```

(3)  The antigenic protein expressed as a polyantigen
containing several FMDV antigens in a continuous
polyprotein.

The FMDV is subject to antigenic drifts and there have now
been identified over 60 different FMDV types and subtypes
(Fernandez, Br. Vet. J. 134, 53 (1978)).  The expression of
several of the $VP_3$ antigens in one long protein has been
investigated.  Such a construction is demonstrated in
pFM20.  This vector expresses, in a stable form, more than
one $VP_3$ antigenic site contained on a single protein.

All of the fragments contained in pFM20 have been described
and consists of 6 DNA fragments:  1, 2, 3, 4a, 5 and 5.

The expression vector expresses, from the trp promoter-operator system, a protein consisting of 190 amino acids coded by the LE' gene construction, linked to a methionine amino acid (CNBr cleavable), linked to the amino acids 1-211 of the $VP_3$ FMDV $A_{12}$ (codons 1-7 are derived from synthetic DNA), linked to amino acids 8-211 of the $VP_3$ FMDV $A_{12}$, linked to 4 amino acids from pBR322 for a total of 609 amino acids.

Table 4

Polyantigen Expression Vector Constructed.

| Expression Vector | DNA Fragments | MW of fusion protein |
|---|---|---|
| pFM20 | 1a, 2, 3, 4a, 5, 5 | 66.8 Kd |

The polyantigen expressed from pFM20.

```
  1                                    10
met lys ala ile phe val leu lys gly ser leu asp arg asp leu
AUG AAA GCA AUU UUC GUA CUG AAA GGU UCA CUG GAC AGA GAU CUC

              20                                      30
asp ser arg ile glu leu glu met arg thr asp his lys glu leu
GAC AGC CGU AUU GAA CUG GAA AUG CGU ACC GAU CAU AAA GAG CUG

                            40
ser glu his leu met leu val asp leu ala arg asn asp leu ala
UCU GAA CAU CUG AUG CUG GUU GAU CUC GCC CGU AAU GAU CUG GCA

              50                                      60
arg ile cys thr pro gly ser arg tyr val ala asp leu thr lys
CGC AUU UGC ACC CCC GGC AGC CGC UAC GUC GCC GAU CUC ACC AAA

                            70
val asp arg tyr ser tyr val met his leu val ser arg val val
GUU GAC CGU UAU UCC UAU GUG AUG CAC CUC GUC UCU CGC GUA GUC

              80                                      90
gly glu leu arg his asp leu asp ala leu his ala tyr arg ala
GGC GAA CUG CGU CAC GAU CUU GAC GCC CUG CAC GCU UAU CGC GCC

                            100
cys met asn met gly thr leu ser gly ala pro lys val arg ala
UGU AUG AAU AUG GGG ACG UUA AGC GGU GCG CCG AAA GUA CGC GCU
```

```
                      110                                              120
met gln leu ile ala glu ala glu gly arg arg arg gly ser tyr
AUG CAG UUA AUU GCC GAG GCG GAA GGU CGU CGC CGC GGC AGC UAC


                      130
gly gly ala val gly tyr phe thr ala his gly asp leu asp thr
GGC GGC GCG GUA GGU UAU UUC ACC GCG CAU GGC GAU CUC GAC ACC


                      140                                              150
cys ile val ile arg ser ala leu val glu asn gly ile ala thr
UGC AUU GUG AUC CGC UCG GCG CUG GUG GAA AAC GGU AUC GCC ACC


                      160
val gln ala gly ala gly val val leu asp ser val pro gln ser
GUG CAA GCG GGU GCU GGU GUA GUC CUU GAU UCU GUU CCG CAG UCG


                      170                                              180
glu ala asp glu thr arg asn lys ala arg ala val leu arg ala
GAA GCC GAC GAA ACC CGU AAC AAA GCC CGC GCU GUA CUG CGC GCU


ile ala thr ala his his ala gln glu phe met thr thr ala thr
AUU GCC ACC GCG CAU CAU GCA CAG GAA UUC AUG ACU ACU GCU ACU
                                      190


                      200                                              210
gly glu ser ala asp pro val thr thr thr val glu asn tyr gly
GGU GAA UCU GCA GAC CCU GUC ACC ACC ACC GUG GAG AAC UAC GGU


                                              220
gly glu thr gln val gln arg arg his his thr asp val ser phe
GGU GAG ACA CAA GUC CAG AGA CGU CAC CAC ACG GAC GUC AGU UUC


                      230                                              240
ile met asp arg phe val lys ile lys ser leu asn pro thr his
AUC AUG GAC AGA UUU GUG AAG AUA AAA AGC UUG AAC CCC ACA CAC


                                      250
val ile asp leu met gln thr his gln his gly leu val gly ala
GUC AUU GAC CUC AUG CAG ACC CAC CAA CAC GGG CUG GUG GGU GCG


                      260                                              270
leu leu arg ala ala thr tyr tyr phe ser asp leu glu ile val
UUG UUG CGU GCA GCC ACG UAC UAC UUC UCC GAC UUG GAG AUU GUU


                                      280
val arg his asp gly asn leu thr trp val pro asn gly ala pro
GUG CGG CAC GAU GGC AAU CUG ACC UGG GUG CCC AAC GGU GCC CCC


                      290                                              300
glu ala ala leu ser asn thr gly asn pro thr ala tyr asn lys
GAG GCA GCC CUG UCA AAC ACC GGC AAC CCC ACU GCC UAC AAC AAG


                                      310
ala pro phe thr arg leu ala leu pro tyr thr ala pro his arg
GCA CCG UUC ACG AGG CUU GCU CUC CCU UAC ACU GCG CCA CAC CGC


                      320                                              330
val leu ala thr val tyr asn gly thr asn lys tyr ser ala ser
GUG UUG GCA ACU GUG UAC AAC GGG ACG AAC AAG UAC UCC GCG AGC


                                      340
gly ser gly val arg gly asp phe gly ser leu ala pro arg val
GGU UCG GGA GUG CGA GGC GAU UUU GGG UCU CUC GCG CCG CGA GUC
```

```
                 350                                        360
ala arg gln leu pro ala ser phe asn tyr gly ala ile lys ala
GCG AGA CAA CUU CCU GCU UCU UUC AAC UAC GGU GCA AUU AAG GCC


                                      370
glu thr ile his glu leu leu val arg met lys arg ala glu leu
GAG ACC AUC CAC GAG CUU CUC GUG CGC AUG AAA CGG GCU GAG CUC


                 380                                        390
tyr cys pro arg pro leu leu ala ile glu val ser ser gln asp
UAC UGC CCC AGG CCA CUG CUG GCA AUA GAG GUG UCU UCG CAA GAC


                                      400
arg his lys gln lys ile ile ala pro gly lys gln asp pro val
AGG CAC AAG CAG AAG AUC AUU GCA CCC GGA AAA CAG GAC CCU GUC


                 410                                        420
thr thr thr val glu asn tyr gly gly glu thr gln val gln arg
ACC ACC ACC GUG GAG AAC UAC GGU GGU GAG ACA CAA GUC CAG AGA


                                      430
arg his his thr asp val ser phe ile met asp arg phe val lys
CGU CAC CAC ACG GAC GUC AGU UUC AUC AUG GAC AGA UUU GUG AAG


                 440                                        450
ile lys ser leu asn pro thr his val ile asp leu met gln thr
AUA AAA AGC UUG AAC CCC ACA CAC GUC AUU GAC CUC AUG CAG ACC


                                      460
his gln his gly leu val gly ala leu leu arg ala ala thr tyr
CAC CAA CAC GGG CUG GUG GGU GCG UUG UUG CGU GCA GCC ACG UAC


                 470                                        480
tyr phe ser asp leu glu ile val val arg his asp gly asn leu
UAC UUC UCC GAC UUG GAG AUU GUU GUG CGG CAC GAU GGC AAU CUG


                                      490
thr trp val pro asn gly ala pro glu ala ala leu ser asn thr
ACC UGG GUG CCC AAC GGU GCC CCC GAG GCA GCC CUG UCA AAC ACC


                 500                                        510
gly asn pro thr ala tyr asn lys ala pro phe thr arg leu ala
GGC AAC CCC ACU GCC UAC AAC AAG GCA CCG UUC ACG AGG CUU GCU


                                      520
leu pro tyr thr ala pro his arg val leu ala thr val tyr asn
CUC CCU UAC ACU GCG CCA CAC CGC GUG UUG GCA ACU GUG UAC AAC


                 530                                       540
gly thr asn lys tyr ser ala ser gly ser gly val arg gly asp
GGG ACG AAC AAG UAC UCC GCG AGC GGU UCG GGA GUG CGA GGC GAU


                                      550
phe gly ser leu ala pro arg val ala arg gln leu pro ala ser
UUU GGG UCU CUC GCG CCG CGA GUC GCG AGA CAA CUU CCU GCU UCU


                 560                                        570
phe asn tyr gly ala ile lys ala glu thr ile his glu leu leu
UUC AAC UAC GGU GCA AUU AAG GCC GAG ACC AUC CAC GAG CUU CUC


                                      580
val arg met lys arg ala glu leu tyr cys pro arg pro leu leu
GUG CGC AUG AAA CGG GCU GAG CUC UAC UGC CCC AGG CCA CUG CUG
```

```
                   590                                         600
ala ile glu val ser ser gln asp arg his lys gln lys ile ile
GCA AUA GAG GUG UCU UCG CAA GAC AGG CAC AAG CAG AAG AUC AUU

                                    609
ala pro gly lys gln asn ser his val OP
GCA CCC GGA AAA CAG AAU UCU CAU GUU UGA
```

Figure 3 shows an SDS PAGE of the total protein from each of the vectors: $pFM_1$, $pFM_2$, $pFM_3$, $pFM_{10}$ and $pFM_{20}$. Lane 1 contains a sample of $VP_3$ from the FMDV, lane 2. E. coli/pBR322, lane 3. E. coli/$pFM_1$, lane 4. E. coli/$pFM_2$, lane 5. E. coli/$pFM_3$, lane 6. E. coli/$pFM_{10}$, lane 7. E. coli/$pFM_{20}$ and lane 8. $VP_3$. Each of these organisms was grown in LB medium then diluted into M9 media as described below.

(4) The antigenic $VP_3$ protein from FMDV $A_{24}$ Cruzeiro Brazil/55 expressed as a fusion protein.

The plasmid construction used to express a fusion protein containing the antigenic sites of the $VP_3$ from strain FMDV $A_{24}$ was the same as that used in $pFM_3$. The coding sequence from most of the $A_{24}$ $VP_3$ gene was joined to the plasmid in place of the corresponding $A_{12}$ $VP_3$ coding sequence. This was accomplished as follows: 1) The plasmid $pFM_3$ was cleaved with HindIII and BamHI. The long DNA fragment containing most of the $tc^R$ gene, the pBR322 origin (most of·DNA fragment 1), the trp promoter and operator (DNA fragment 2), the short LE' fusion gene (DNA fragment 3a), the synthetic $VP_3$ gene fragment (DNA fragment 4a) and codons 10-44 of the FMDV $A_{12}$ $VP_3$ gene, was isolated by polyacrylamide gel electrophoresis. The plasmid insert B435 (see Figure 1) was digested with HindIII and partially digested with HaeIII to give a 533 base pair DNA fragment from the FMDV $A_{24}$ $VP_3$ gene
coding for amino acids 44-220 (about 7 beyond the end of
the $VP_3$ gene). This was isolated by polyacrylamide gel.
3) The plasmid pBR322 (fragment 1) was digested with EcoRI,
treated with DNA polymerase I Klenow fragment, to fill in
the EcoRI end, then treated with BamHI. The 375 base pair
fragment was isolated by polyacrylamide gel. 4) The three
fragments (1, 2 and 3) were combined, joined with T4 DNA
ligase and the mixture used to transform E. coli to $tc^R$.
The identical plasmid was isolated from several colonies
and shown to contain the correct arrangment of DNA
fragments.

The amino acid sequence of the expressed protein is shown
below as deduced from the nucleotide sequence.

Fusion protein expressed from pFMBI (FMDV $A_{24}$).

```
 1                                  10
met lys ala ile phe val leu lys gly ser leu asp arg asp pro
AUG AAA GCA AUU UUC GUA CUG AAA GGU UCA CUG GAC AGA GAU CCA

              20                                  30
glu phe met thr thr ala thr gly glu ser ala asp pro val thr
GAA UUC AUG ACU ACU GCU ACU GGU GAA UCU GCA GAC CCU GUC ACC

                        40
thr thr val glu asn tyr gly gly glu thr gln val gln arg arg
ACC ACC GUG GAG AAC UAC GGU GGU GAG ACA CAA GUC CAG AGA CGU

              50                                  60
his his thr asp val ser phe ile met asp arg phe val lys ile
CAC CAC ACG GAC GUC AGU UUC AUC AUG GAC AGA UUU GUG AAG AUA

                        70
AAA AGC UUG AGC CCA ACA CAU GUC AUU GAC CUC AUG CAG ACU CAC
lys ser leu ser pro thr his val ile asp leu met gln thr his

              80                                  90
gln his gly leu val gly ala leu leu arg ala ala thr tyr tyr
CAA CAC GGU CUG GUG GGU GCC UUG CUG CGU GCA GCC ACG UAC UAC

                        100
phe ser asp leu glu ile val val arg his glu gly asn leu thr
UUU UCU GAC UUG GAA AUU GUU GUA CGG CAC GAA GGC AAU CUG ACC
```

```
                        110                                    120
trp val pro asn gly ala pro glu ser ala leu leu asn thr ser
UGG GUG CCC AAC GGC GCC CCU GAA UCA GCC CUG UUG AAC ACC AGC

                                          130
asn pro thr ala tyr asn lys ala pro phe thr arg leu ala leu
AAC CCC ACU GCC UAC AAC AAG GCA CCA UUC ACG AGA CUC GCU CUC

                        140                                    150
pro tyr thr ala pro his arg val ser ala thr val tyr asn gly
CCC UAC ACU GCG CCG CAC CGU GUG UCG GCA ACA GUG UAC AAC GGG

                                          160
thr ser lys tyr ala val gly gly ser gly arg arg gly asp met
ACG AGU AAG UAU GCU GUG GGU GGU UCA GGC AGA CGU GGC GAC AUG

                        170                                    180
gly thr leu ala ala arg val val lys gln leu pro ala ser phe
GGG ACU CUC GCG GCG CGA GUC GUG AAA CAG CUU CCU GCU UCA UUU

                                          190
asn tyr gly ala ile lys ala asp ala ile his glu leu leu val
AAC UAC GGU GCA AUC AAG GCC GAC GCC AUC CAC GAA CUU CUC GUG

                        200                                    210
arg met lys arg ala glu leu tyr cys pro arg pro leu leu ala
CGC AUG AAA CGG GCC GAG CUC UAC UGC CCC AGA CCG CUG CUG GCA

                                          220
ile glu val ser ser gln asp arg his lys gln lys ile ile ala
AUA GAG GUG UCU UCG CAA GAC AGG CAC AAG CAA AAG AUC AUU GCA

                        230                                    240
pro ala lys gln leu leu asn phe asp leu leu lys leu glu phe
CCA GCA AAG CAG CUU CUG AAU UUU GAC CUG CUC AAG UUG GAA UUC

                          248
ser cys leu thr ala tyr his arg OC
UCA UGU UUG ACA GCU UAU CAU CGA UAA
```

(5)  The antigenic VP$_3$ protein from FMDV A$_{27}$
Cundinamarca Columbia/76 expressed as a fusion protein.


The plasmid construction used to express a fusion protein
containing the antigenic sites of the VP$_3$ from strain
FMDV A$_{27}$ was the same as that used in pFM$_3$.  The coding
sequence for the A$_{27}$ gene was obtained from the insert
C312 (see Figure 1) in two DNA fragments.  The first part
of the gene was obtained by treating the plasmid insert
C312 with PstI then isolating the 900 base pair insert.
This fragment was combined and annealed with a synthetic oligonucleotide, $^{32}$P CATGACCACTGTCA, complementary to
this first three codons of the $VP_3$ gene and adding a .
synthetic met codon prior to the gene. The primer was
elongated with DNA polymerase Klenow fragment and made
blunt ended at the primer due to the 3' to 5' exonuclease
and repair activities of the polymerase using the procedure
described by Goeddel et al., Nucleic Acids Research 8,
4057-4074 (1980). The mixture was treated with SacI and a
580 base pair radiolabelled fragment isolated by
polyacrylamide gel electrophoresis. This DNA fragment
contains CATG followed by the first 182 codons of the FMDV
$A_{27}$ $VP_3$ gene.

The second part of the gene was isolated from insert C312
by treatment with SacI and EcoRI under low salt conditions
such that the sequence, GAATTT (an EcoRI* site) at the
final codon of the $VP_3$ gene, was cleaved. The 90 base
pair fragment was isolated by polyacrylamide gel
electrophoresis. This fragment was ligated into pBR322
previously treated with PstI and EcoRI and the large
fragment (DNA fragment 1) isolated. The 90 base pair
fragment was combined with a PstI-SacI DNA fragment
obtained from C312. The ligation, followed by
transformation into E. coli, gave a plasmid, $pBRA_{27}$.
From this plasmid a SacI to BamHI fragment was isolated
containing the last 30 codons of the FMDV $A_{27}$ $VP_3$ gene
connected to 375 base pairs of pBR322 containing the
beginning of the tc$^R$ gene. This DNA fragment was
combined with the first part of the $VP_3$ gene, isolated
above using the primer reaction, and this combined with the
expression vector. The expression vector, derived from
$pFM_3$ contains most of the tc$^R$ gene, the pBR322 origin (most of DNA fragment 1), the trp promoter and operator
(DNA fragment 2), and the short LE' fusion (DNA fragment
3a). The vector was previously treated with EcoRI and DNA
polymerase I to create a blunt end at the end of the short
LE' fusion (see sequence of fragment 3a), followed by
treatment with BamHI and isolation by polyacrylamide gel
electrophoresis. The three DNA fragments were treated with
T4 DNA ligase and used to transform E. coli. The plasmid
pFMC was isolated and shown to contain the correct
arrangement of fragments.

The amino acid sequence of the expressed protein is shown
below as deduced from the nucleotide sequence.

Fusion protein expressed from pFMC (FMDV $A_{27}$).

```
 1                                        10
met lys ala ile phe val leu lys gly ser leu asp arg asp pro
AUG AAA GCA AUU UUC GUA CUG AAA GGU UCA CUG GAC AGA GAU CCA

                         20                               30
.glu phe met thr thr val thr gly glu ser ala asp pro val thr
 GAA UUC AUG ACC ACU GUC ACU GGG GAG UCC GCA GAC CCU GUC ACA

                                         40
thr thr val glu asn tyr gly gly glu thr gln val gln arg arg
ACC ACC GUG GAG AAC UAC GGC GGU GAG ACA CAA GUC CAA AGA CGG

                         50                               60
his his thr asp val gly phe ile met asp arg phe val lys ile
CAC CAC ACG GAU GUC GGG UUC AUU AUG GAC AGA UUU GUG AAA AUA

                                         70
asn asn leu ser pro thr his val ile asp leu met gln thr his
AAC AAU CUG AGU CCC ACA CAU GUC AUU GAC CUC AUG CAG ACC CAC

                         80                               90
gln his gly leu val gly ala leu leu arg ala ala thr tyr tyr
CAG CAC GGG UUG GUA GGU GCG CUG UUG CGU GCA GCC ACC UAC UAC

                                        100
phe ser asp leu glu ile val val arg his asp gly asn leu thr
UUC UCU GAC CUU GAG AUU GUU GUG CGC CAC GAC GGC AAC CUG ACU

                        110                              120
trp val pro asn gly ala pro glu ala ala leu ser asn thr ser
UGG GUG CCC AAC GGU GCC CCC GAA GCA GCC CUC UCA AAC ACC AGC
```

```
                                   130
asn pro thr ala tyr asn lys ala pro phe thr arg leu ala leu
AAC CCC ACU GCC UAC AAC AAG GCA CCA UUC ACG AGA CUC GCU CUC

            140                                       150
pro tyr thr ala pro his arg val leu ala thr val tyr asn phe
CCG UAC ACC GCG CCA CAC CGU GUG UUG GCA ACC GUG UAC AAC UUU

                                   160
thr asn lys tyr ser asn gly gly gln arg ala gly asp met gly
ACG AAC AAG UAC UCC AAC GGU GGC CAG AGG GCA GGU GAC AUG GGG

            170                                       180
ser leu ala ala arg val ala lys gln leu pro ala ser phe asn
UCA CUU GCG GCA CGG GUC GCA AAA CAG CUU CCU GCU UCU UUC AAC

                                   190
tyr gly ala ile lys ala gln thr ile his glu leu leu val arg
UAC GGU GCA AUC AAG GCC CAG ACC AUC CAC GAG CUU CUC GUG CGC

            200                                       210
met lys arg ala glu leu tyr cys pro arg pro leu leu ala ile
AUG AAA AGG GCG GAG CUC UAC UGC CCC AGA CCA CUG UUG GCA AUA

                                   220
glu val ser ser gln asp arg his lys gln lys ile ile ala pro
GAG GUG UCU UCG CAA GAC AGG CAC AAG CAG AAG AUC AUU GCG CCC

            230           234
ala lys gln leu leu asn ser his val OP
GCA AAG CAG UUG UUG AAU UCU CAU GUU UGA
```

(6)  The antigenic VP3 protein from FMDV A Argentina/79,
expressed as a fusion protein.

The plasmid construction used to express a fusion protein
containing the antigenic sites of the VP3 from strain
FMDV A Argentina/79 was the same as that used in pFM3.
The coding sequence for the A Argentina/79 gene was
obtained from the insert F64 (see Table 1), again in two
DNA fragments.  The first part of the gene was obtained by
the same primer extension, SacI cleavage reaction used for
A27.  The primer used in A Argentina/79 was $^{32}$P
CATGACTACCGCCA and the 522 base pair fragment obtained was
ligated into pFM3 previously treated with EcoRI, DNA
polymerase I, and BamHI as described for pFMC.  These two
fragments were combined with a SacI-BamHI fragment from

pFMB containing the last 100 base pairs of the $A_{24}$ $VP_3$ gene and the first 375 base pairs of the $Tc^R$ gene. The three joined fragments were used to transform E. coli to $Tc^R$ and from this the plasmid pFMF-1 obtained. In order to reconstruct the complete coding sequence of the A Argentina/79 $VP_3$ gene, this plasmid was cleaved with SacI and HindIII and the large fragment isolated by polyacrylamide gel electrophoresis. This fragment was combined with two more fragments, a SacI-BstN1 40 base pair fragment containing codons 174 to 186 of the A Argentina/79 and a BstN1-HindIII 105 base pair fragment containing codons 187 to 211 of the $A_{12}$ $VP_3$ gene and the pBR322 sequence from EcoRI to HindIII. These three fragments were joined by ligation and used to transform E. coli.

The amino acid sequence of the protein designed to be expressed from this construction, as deduced from the nucleotide sequence, is shown below.

Fusion protein designed to be expressed from the FMDV A Argentina/79 strain.

```
 1                                   10
met lys ala ile phe val leu lys gly ser leu asp arg asp pro
AUG AAA GCA AUU UUC GUA CUG AAA GGU UCA CUG GAC AGA GAU CCA

                20                                   30
glu phe met thr thr ala thr gly glu ser ala asp pro val thr
GAA UUC AUG ACU ACC GCC ACC GGG GAG UCA GCA GAC CCU GUU ACC

                                    40
thr thr val glu asn tyr gly gly glu thr gln val gln arg arg
ACC ACC GUG GAG AAC UAC GGC GGU GAG ACA CAG GUU CAG AGA CGC

                50                                   60
tyr his thr asp ile gly phe ile met asp arg phe val lys ile
UAC CAC ACU GAC AUC GGC UUC AUC AUG GAC AGG UUU GUG AAG AUU
```

```
                                        70
lys asp val gln pro thr his val ile asp leu met gln thr his
AAG GAC GUG CAA CCG ACG CAU GUC AUU GAC CUU AUG CAG ACU CAC

                    80                                   90
gln tyr gly leu val gly ala ile leu arg ala ala thr tyr tyr
CAA UAC GGC CUA GUG GGU GCA AUC CUG CGU GCA GCU ACG UAC UAC

                                   100
phe ser asp leu glu ile val val arg his asp gly asn leu thr
UUU UCU GAC UUG GAA AUU GUU GUA CGG CAC GAC GGC AAC CUG ACU

                110                                  120
leu val pro asn gly ala pro glu ser ala leu asp asn thr gly
UUG GUG CCC AAC GGC GCC CCU GAG UCA GCC CUA GAC AAC ACU GGC

                                   130
asn pro thr ala tyr asn lys ala pro phe thr arg leu ala leu
AAU CCC ACC GCC UAC AAC AAG GCA CCA UUC ACG AGA CUU GCU CUC

                    140                                  150
pro tyr thr ala pro his arg val leu ala thr val tyr asn gly
CCU UAC ACG GCA CCA CAC CGU GUG CUG GCA ACA GUG UAC AAC GGG

                                   160
thr ser lys tyr thr val gly gly ser gly arg arg gly asp met
ACA AGC AAA UAC ACC GUG GGU GGU UCA GGC AGG CGU GGU GAC AUG

                170                                  180
gly ser leu ala ala arg val ala lys gln leu pro ala ser phe
GGG UCC CUC GCG GCA CGA GUC GCG AAA CAA CUU CCU GCU UCA UUC

                                   190
asn tyr gly ala ile lys ala thr ala ile his glu leu ile val
AAC UAU GGU GCA AUU AAG GCC ACC GCC AUC CAC GAG CUC AUC GUG

                    200                                  210
arg met lys arg ala glu leu tyr cys pro arg pro leu leu ala
CGC AUG AAA CGG GCC GAA CUC UAC UGU CCC AGG CCA CUG CUG GCA

                                   220
ile glu val ser ser gln asp arg his lys gln lys ile ile ala
AUA GAG GUG UCU UCG CAA GAC AGG CAC AAG CAG AAG AUC AUU GCA

                230         233
pro gly lys gln asn ser his val OP
CCC GGA AAA CAG AAU UCU CAU GUU UGA
```

(7) The antigenic VP₃ protein from FMDV C₃ Resende
Brazil/55 expressed as a fusion protein.


The plasmid construction used to express a fusion protein
containing the antigenic sites of the VP₃ from strain
FMDV C₃ Resende Brazil/55 was the same as that used in
pFM₃. The coding sequence for the C₃ Resende VP₃ gene was obtained from the insert D250 (see Table 1) again
in two DNA fragments.  The first part of the gene was
obtained by the same primer extension reaction described
for $A_{27}$ using the primer $^{32}$P CATGACCACTG.  This primer
codes for an initial C followed by codon two (which is a
met) through five of the $C_3$ $VP_3$ gene.  Following the
primer extension reaction, the DNA mixture was treated with
BstEII to give a 240 base pair fragment containing the
second through the 75th codon of the $VP_3$ gene.  To obtain
the second part of the gene the insert D250 was cleaved
with Pst and ThaI.  The ThaI cleaves at codon 204 of the
$C_3$ $VP_3$ gene.  This fragment was joined to pBR322
previously treated with EcoRI, DNA polymerase, and PstI and
used to transform E. coli.  By treating the resulting
plasmid with BstEII and BamHI a DNA fragment containing the
codons 75-205 of the $C_3$ $VP_3$ gene connected to the first
375 bp of the pBR322 was obtained.  This latter fragment
was combined with the product of the primer extension
reaction and joined to the vector.  The vector was the same
fragment used in the construction of pFMC and pFMF-1
described above.  These joined fragments were used to
transform E. coli to $Tc^R$.  The plasmid pFMD was isolated
and shown to contain the correct arrangement of fragments.

The amino acid sequence of the expressed protein is shown
below as deduced from the nucleotide sequence.

Fusion protein expressed from pFMD (FMDV $C_3$).

```
1                                    10
met lys ala ile phe val leu lys gly ser leu asp arg asp pro
AUG AAA GCA AUU UUC GUA CUG AAA GGU UCA CUG GAC AGA GAU CCA
```

```
                    20                                          30
glu phe met thr thr gly glu ser ala asp pro val thr thr thr
GAA UUC AUG ACC ACU GGU GAA UCU GCC GAC CCU GUU ACC ACU ACC


                                        40
val glu asn tyr gly gly glu thr gln val gln arg arg his his
GUU GAG AAC UAC GGA GGA GAG ACG CAA GUU CAA CGU CGU CAC CAC


                    50                                          60
thr asp val ala phe val leu asp arg phe val lys val pro val
ACU GAC GUU GCC UUC GUU CUU GAC CGG UUU GUG AAG GUC CCU GUG


                                        70
ser asp gly gln gln his thr leu asp val met gln val his lys
UCG GAC GGA CAA CAA CAC ACA CUG GAC GUG AUG CAG GUA CAC AAG


                    80                                          90
asp ser ile val gly ala leu leu arg ala ala thr tyr tyr phe
GAC AGU AUC GUG GGA GCG CUU CUC CGC GCA GCC ACG UAC UAC UUC


                                        100
ser asp leu glu ile ala val thr his thr gly lys leu thr trp
UCU GAU CUG GAA AUA GCG GUG ACC CAC ACC GGG AAG CUC ACC UGG


                    110                                         120
val pro asn gly ala pro val ser ala leu asp asn thr thr asn
GUG CCC AAC GGU GCA CCG GUU UCU GCA CUU GAC AAC ACA ACC AAC


                                        130
pro thr ala tyr his lys arg pro leu thr arg leu ala leu pro
CCC ACU GCA UAC CAC AAG AGA CCG CUG ACU CGA CUG GCU CUC CCA


                    140                                         150
tyr thr ala pro his arg val leu ala thr thr tyr thr gly thr
UAC ACC GCG CCA CAC CGC GUG UUG GCC ACG ACG UAC ACU GGU ACA


                                        160
thr thr tyr thr thr ser ala arg arg gly asp leu val his leu
ACA ACC UAC ACU ACC AGU GCA CGU AGA GGA GAU UUG GUC CAU UUG


                    170                                         180
ala ala ala his ala arg his leu pro thr ser phe asn phe gly
GCG GCA GCA CAC GCU CGG CAC UUG CCG ACG UCG UUC AAC UUU GGU


                                        190
ala val lys ala glu lys val thr glu leu leu val arg met lys
GCA GUU AAA GCA GAA AAA GUC ACU GAG CUG CUG GUG CGC AUG AAG


                    200                                         210
arg ala glu leu tyr cys pro arg pro ile pro pro ile arg pro
CGU GCA GAA CUC UAU UGC CCC AGG CCG AUU CCU CCG AUU CGG CCA


                                        220
thr gly asp arg his lys gln ser phe ile glu phe ser cys leu
ACG GGC GAC AGA CAC AAG CAA UCG UUU AUC GAA UUC UCA UGU UUG


                    230
thr ala tyr his arg OC
ACA GCU UAU CAU CGA UAA
```

(8) The antigenic VP$_3$ protein from FMDV O$_1$ Campos Brazil/58 expressed as a fusion protein.

The plasmid construction used to express a fusion protein containing the antigenic sites of the VP$_3$ from strain FMDV O$_1$ Campos Brazil/58 was the same as that used in pFM$_3$. The coding sequence for the O$_1$ Campos VP$_3$ gene was obtained from the insert G85 (see Table 1) by treatment with BamHI and EcoRI under low salt conditions such that the GAATTT EcoRI* site is cleaved. The 620 base pair fragment isolated contained codons 8 through 213 of the VP$_3$ gene. The PstI site in the vector pFM$_3$ at codon 8 was converted to a BglII site. This was done by ligating the synthetic oligonucleotide 5' GATCTGCA to the Pst-cleaved plasmid followed by treatment with BglII. The plasmid was then cleaved with AvaI to give the pBR322 origin of replication, the trp operator and promoter (DNA fragment 2), the short LE' fusion (DNA fragment 3a), the synthetic codons of the FMDV A$_{12}$ gene (DNA fragment 4a) and terminating in a sequence suitable for ligation of a BamHI end. When joined to the 620 base pair fragment isolated from the G85 insert, and a 1424 base pair fragment obtained by digesting plasmid pBR322 with EcoRI and AvaI, this construction recreates the natural O$_1$ Campos VP$_3$ sequence from codons 5 through 213. Thus the vector and the O$_1$ Campos VP$_3$ coding sequence were joined by ligation and used to transform E. coli to Tc$^R$. The plasmid pFMG was isolated and shown to contain the correct arrangement of fragments.

The amino acid sequence of the expressed protein is shown below as deduced from the nucleotide sequence.

Fusion protein expressed from pFMG (FMDV $O_1$).

```
 1                                      10
met lys ala ile phe val leu lys gly ser leu asp arg asp pro
AUG AAA GCA AUU UUC GUA CUG AAA GGU UCA CUG GAC AGA GAU CCA


                   20                                      30
glu phe met thr thr ala thr gly glu ser ala asp pro val thr
GAA UUC AUG ACU ACU GCU ACU GGU GAA UCU GCA GAU CCU GUC ACC


                                       40
ala thr val glu asn tyr gly gly glu thr gln ile gln arg arg
GCC ACU GUU GAA AAC UAC GGU GGC GAA ACA CAG AUC CAG AGG CGC


                   50                            -           60
gln his thr asp val ser phe ile met asp arg phe val lys val
CAA CAC ACG GAC GUC UCG UUC AUC AUG GAC AGA UUU GUG AAA GUG


                                       70
thr pro gln asn gln ile asn ile leu asp leu met gln ile pro
ACA CCG CAA AAC CAA AUU AAC AUU UUG GAC CUC AUG CAG AUU CCA


                   80                                      90
ser his thr leu val gly ala leu leu arg ala ser thr tyr tyr
UCA CAC ACU UUG GUG GGA GCG CUC CUA CGC GCG UCC ACU UAC UAC


                                       100
phe ser asp leu glu ile ala val lys his glu gly asp leu thr
UUC UCU GAC UUG GAG AUA GCA GUA AAA CAC GAG GGA GAC CUC ACC


                   110                                     120
trp val pro asn gly ala pro glu lys ala leu asp asn thr thr
UGG GUU CCA AAU GGA GCG CCU GAA AAG GCG UUG GAC AAC ACC ACC


                                       130
asn pro thr ala tyr his lys ala pro leu thr arg leu ala leu
AAC CCA ACU GCU UAC CAC AAG GCA CCA CUC ACC CGG CUU GCC CUG


                   140                                     150
pro tyr thr ala pro his arg val leu ala thr val tyr asn gly
CCC UAC ACC GCG CCC CAC CGC GUG UUG GCA ACC GUG UAC AAC GGU


                                       160
glu cys arg tyr ser arg asn ala val pro asn val arg gly asp
GAG UGC AGG UAC AGC AGA AAU GCU GUG CCC AAC GUG AGA GGU GAC


                   170                                     180
leu gln val leu ala gln lys val ala arg thr leu pro thr ser
CUU CAG GUG UUG GCU CAA AAG GUG GCA CGG ACG CUG CCU ACC UCC


                                       190
phe asn tyr gly ala ile lys ala thr arg val thr glu leu leu
UUC AAC UAC GGU GCC AUC AAA GCG ACC CGG GUC ACC GAG UUG CUU


                   200                                     210
tyr arg met lys arg ala glu thr tyr cys pro arg pro leu leu
UAC CGG AUG AAG AGG GCC GAA ACA UAC UGU CCA AGG CCC UUG CUG


                                       220
ala ile his pro thr glu ala arg his lys gln lys ile val ala
GCA AUC CAC CCA ACU GAA GCC AGA CAC AAA CAG AAA AUU GUG GCA
```

```
                    230                    235
pro val lys gln thr leu asn ser his val OP
CCG GUG AAA CAG ACU UUG AAU UCU CAU GUU UGA
```

U.  Isolation of Antigenic Proteins from E. coli K12 containing
Expression Vectors.

The expression vectors described above have been
incorporated into E. coli hosts, e.g. K-12 294, ATCC No.
31446, K-12 RV 308, ATCC No. 31608, and K-12 W3110, ATCC
No. 27325.

(1)  The isolation and properties of the fusion protein
expressed from the E. coli K12 294/pFM1 are presented here
in detail.

The recombinant E. coli was grown to $A_{550}$ = 1 in LB medium
(Miller, J.H., Experiments in Molecular Genetics, Cold
Spring Harbor, NY, 1972) containing 5 μg/ml tetracycline.
The culture (40 ml) was diluted into 800 ml of M9 media
(ibid) containing, per liter, 1 ml of 1 M $MgSO_4$, 25 ml of
20 percent glucose, 25 ml of 20 percent casamino acids and
0.1 ml of 1 percent thiamine.  This was grown at 37°C to
$A_{550}$ = 2 and the bacteria collected by centrifugation, at
5000 rpm for 15 min. at 4°.  The bacteria were resuspended
in 80 ml of 50 mM Tris HCl pH 7.5, 0.5 mM EDTA, 0.3 M NaCl
(TEN) then treated on ice for 15 min with lysozyme (1
mg/ml) and made 0.2 percent in NP-40.  After 10 min the
sample was diluted to 200 ml and made 0.9 M in NaCl then
treated with DNase (0.002 mg/ml final concentration) and
$MgCl_2$ (5 mM final concentration) for 10 min.  The
precipitated protein was recovered by centrifugation (5000
rpm for 15 min. at 4°) and resuspended and washed with 30 .
ml TEN three times.  The precipitated protein totaled 450 mg by Lowery assay (approximately 45 percent of the total protein) and much of this (170 mg, approximately 17 percent of the total protein) was the LE'-VP$_3$ fusion protein as determined by P.A.G.E. analytical scanning.

Figure 4 shows an SDS P.A.G.E. analysis of the LE'-VP$_3$ fusion protein. Each sample is shown in pairs, the first lane of the pair shows the total bacterial protein stained with Coomassie blue and the second lane of the pair shows an experiment where the protein was transferred to CNBr treated paper and exposed to $I^{125}$ labelled antibody to VP$_3$.

This was carried out using a modification of the colony screening method of Hitzeman et al. in Eucaryotic Gene Regulation 14, 57-68 [Academic Press, N.Y. (1979)]. The polyacrylamide slab gel 1.27 mm thick 9x14 cm of 12.5 percent acrylamide containing 8 M urea (Maizel, J.V. in Methods in Virology Vol. 5, pp 179-246, Academic Press, NY) was washed 30 min in 2 changes of phosphate buffered saline (PBS) solution. This was placed on CNBr activated paper (Hitzeman et al., J. Biol. Chem. 255, 12073-12080 (1980)), for one hour at room temperature. The gel was later stained using Coomassie blue. The paper was then treated in PBS containing 0.1 M glycine, and 0.2 percent BSA at 37°C for 4 hrs (alternatively 18 hrs at 4°C), then washed in PBS for 10 min. The paper was then placed in a plastic bag and treated for 1 hr at 37°C with 5 percent guinea pig serum in PBS. This was drained and 7.5x10$^6$ (up to 10x10$^6$) cpm $^{125}$I-guinea pig IgG specific for VP$_3$ in 10 ml 5 percent guinea pig serum in PBS was added. (The VP$_3$ specific antibody was iodinated using 100 µg IgG, 10 µg

Iodogen, 500 µCi $^{125}$I (Na I) for 15 min at room temperature, followed by G25 sephadex chromatography). After 4 hrs at room temperature the filter was washed with 30 ml of 5 percent guinea pig serum in PBS for 1 hr, then rinsed 2 times with 200 ml PBS for 18 hr. The filter was blotted and air dried then exposed to X-ray film using an intensifying screen at -60°C.

Figure 4 shows in lanes 1, 1a E. coli 294, lanes 2, 2a E. coli 294/pFM control. (The FMDV $VP_3$ sequences are attached to the expression vector in the incorrect reading frame), lanes 3, 3a $pFM_1$ partially induced for fusion protein expression (not grown up such that the tryptophan operon was completely induced), lanes 4, 4a $pFM_1$ fully induced, lanes 5, 5a FMDV $A_{12}$ 119 ab $VP_1$, $VP_2$, $VP_3$ (140S capsids).

The fusion protein was purified two times by SDS P.A.G.E. using a 1 cm thick 10 percent slab gel in 8 M urea [H.L. Bachrach et al., Virology 52, 520-528 (1973); H.D. Matheka and H.L. Bachrach, J. Virology 16, 1248-1253 (1975)]. The protein containing bands were visualized by chilling the gel [H.L. Bachrach, Analyt. Biochem 110, 349-359 (1981)] and the bands were cut out and protein recovered by electroelution or pulverization (Braatz, J.A., and McIntire, K.P., Prep. Biochem 7, 495-509 (1972)]. The PAGE gel slurry was taken up in Tris-glycine buffer pH 8.3 and 0.1 percent SDS containing 0.05 M ß-mercaptoethanol. This was heated to 100°C for 5 min to insure no contamination of infectious FMDV viral particles.

(2) The isolation of fusion protein expressed from the E. coli K12 W3110/pFM3 is presented here in detail.

Approximately 240 gms of wet cell paste (cells grown in a 10 liter fermentor) were suspended in 2 liters of Sucrose Lysis Buffer (SLB: 10 percent Sucrose, 50 mM Tris, 0.2 M NaCl, 100 mM EDTA, pH = 7.8). Lysozyme (1 mg/ml) was added to the suspension. This was sonicated for three 9 minute periods in a stainless steel beaker cooled with an ice bath. Centrifugation at 12 K RPM for 40 minutes at 4°C yielded 80 grams of wet pellet which was resuspended in 1.5 liters Urea-8 buffer (8.0 M Urea, 0.014 M Tris-HCl, 1.0 percent βME at pH 8.4). (The sonication step can be replaced with a Manton-Gaulin milling procedure.) The solution was stirred for 2-1/2 hours at 4°C with 375 g DE52 Cellulose previously equilibrated with Urea-6 buffer (6.0 M Urea, 0.014 M Tris-HCl, 1.0 percent βME at pH 8.4). After filtration the supernatant was combined with 1 kg of previously equilibrated CM52 Cellulose (in Urea-6 buffer) and after 3 hours the supernatant was collected. The resin was washed with an additional liter of Urea-6 buffer.

The resin was resuspended in 1 liter of Urea-6 buffer 0.4 M in NaCl, then stirred ~12 hours at 4°C and the resulting supernatant collected. The supernatant was concentrated from 1.0 liter to 100 ml on a YM10 Amicon membrane. A sample was dialyzed versus Urea 6 buffer to remove NaCl and applied to a CM52 column (~2.2 x 24 cm) equilibrated with Urea-6 buffer. The column was eluted with 0 to 0.1 M NaCl gradient (in Urea-6 buffer) and the fractions containing the fusion protein were combined. This material was greater than 90 percent pure as judged by SDS polyacrylamide gel electrophoresis.

E.  Antigenicity of the Fusion Protein Expressed from pFM₁

The fusion protein from pFM₁ (LE'-VP₃) was compared by radioimmunoassay with the purified virion protein VP₃ for ability to compete with $^{125}$I-labelled 140S virion capsids for VP₃ specific antibody.  The VP₃ protein or the fusion protein was taken up in 6 M urea, 0.1 M β-mercaptoethanol and 0.014 Tris-HCl pH 8.6.  The amount of protein added to each assay varied from 30 to 0.2 femtomoles (see Table 5 below).  The assay solution consisted of 0.4 ml guinea pig antiserum for VP₃ in an isotonic borate buffered saline solution.  Per liter this solution contains 7.4 gm Boric Acid, 7.6 gm sodium tetraborate, 4.38 gm NaCl, and is 0.005 percent sodium azide, 0.001 M KI, 0.02 percent BSA at pH 8.1).  After 1 hr at room temperature 0.1 ml of a solution containing $^{125}$I-labelled 140S capsids (these molecules comprise the VP₁, VP₂ and VP₃ proteins of FMDV) was added.  After 1 hr at room temperature Staphylococcus cells (0.2 ml of a 1 percent suspension in borate buffer) was added to precipitate the antibody-antigen complex (Kessler, S.W., J. Immun. 177, 1482-1490 (1976)).  After 15 min at room temperature the mixture was centrifuged, the supernatant removed and the radioactivity in the pellet determined.  Table 5 and Figure 5 show the results of this analysis.  From this data it appears that the fusion protein (LE'-VP₃) and the VP₃ protein possess similar antigenic properties in this competition assay.

Table 5

A comparison of $VP_3$ vs. the $pFM_1$ fusion protein's ($LE'-VP_3$) ability to inhibit the guinea pig anti-$VP_3$-140S FMDV capsid protein reaction in a competitive radioimmunoassay. Fm is femtomoles of antigen added to the $^{125}I$-complex. Percent max. is the percent of labeled 140S capsids associated with the $VP_3$ specific antibody.

| $LE'-VP_3$ | | $VP_3$ | |
|------|----------------|------|----------------|
| fm | Percent max. | fm | Percent max. |
| 27.5 | 10.2 | 37.3 | 4.9 |
| 13.7 | 16.5 | 18.7 | 11.7 |
| 6.9 | 28.6 | 9.3 | 20.4 |
| 2.73 | 80.4 | 3.73 | 57.1 |
| 1.37 | 100.1 | 1.87 | 94.7 |
| 0.68 | 100.2 | 0.93 | 96.7 |
| 0.27 | 100.6 | 0.37 | 97.0 |

F. Vaccine Preparation with fusion protein from pFM1 [see D(1.)]

The vaccine was prepared as a 50:50 oil adjuvant:aqueous emulsion. One dose of test vaccine consisted of 3.5 ml of gel slurry material containing 250 µg of $LE'-VP_3$ (from $pFM_1$) combined with 3.5 ml incomplete Freund-type oil (Marcol 52 oil, Arlacel A 10 percent, Emulugin 05 1 percent). The positive control vaccine contained 140 µg of purified $VP_3$ in the same 50:50 emulsion described above.

G. Vaccine Preparation with fusion protein from pFM3 [see D(2.)]

The vaccine was prepared as described above in 50:50 oil adjuvant:aqueous emulsion using the purified fusion protein from pFM3 dissolved in 8M urea. The vaccine was prepared in different dosage forms: 10 μg protein per sample, 50 μg protein per sample, 250 μg protein per sample and 1250 μg protein per sample.

H. Innocuity Test

The LE'-VP$_3$ vaccine and samples of the purified VP$_3$ vaccine were tested to insure that the test vaccines did not contain any virulent FMD virus. Samples of the inoculant were safety tested in 6 susceptible bovine by the method of Henderson (J. Hyg. 50, 182-192 (1952)). Each bovine was inoculated intradermalingually at 20 sites with a total of 2 ml of inoculum. These animals were kept and observed for evidence of FMD for 14 days.

I. Serology of animals vaccinated with fusion protein from pFM1 [F.]

Serum samples of 6 bovine and 2 porcine inoculated with LE'-VP$_3$ (250 μg in 7 ml 50:50 aqueous:oil adjuvant) and 2 bovine inoculated with VP$_3$ (140 μg in 7 ml of 50:50 aqueous-oil adjuvant) were collected at day 7, 14, 28, 35 and 42 post vaccination. The animals were revaccinated on the 28th day. The titers of the neutralizing FMDV antibodies in these serums was determined by the suckling mice protection assay of Skinner [Proceedings 15th International Veterinary Congress, Part 1, Vol. 1, p. 195 (1952)]. The neutralizing antibody titers (PD$_{50}$) are expressed as the log of the reciprocal of the dilution calculated to protect 50 percent of the mice. That is, if a 1:1000 dilution of the serum protects 50 percent of the mice the titer is 3.0. Inoculants were prepared each containing 200 LD$_{50}$

units of virus combined with an equal volume of the various serum dilutions. 0.03 ml of these inoculants were used for injecting the animals.

Table 6

Serological results of vaccinated animals.

| Bovine No. | 7 | 14 | 21 | 28 | 35 | 42 | days |
|---|---|---|---|---|---|---|---|
| 98 | <.30 | .47 | .30 | 1.17 | 2.31 | 2.66 | |
| 99 | .21 | .91 | 1.26 | 1.96 | 2.40 | 2.66 | |
| 101 | .21 | .39 | 1.09 | 1.70 | 1.88 | 2.05 | |
| 102 | .21 | .65 | .65 | 1.09 | 2.23 | 2.57 | |
| 104 | <.30 | .91 | 1.00 | 1.09 | 2.57 | 2.49 | |
| 105 | .04 | 1.00 | 1.26 | 1.26 | 2.05 | 2.05 | |
| 100 * | .13 | 1.00 | 2.05 | 1.87 | 2.23 | 2.57 | |
| 103 * | <.30 | .39 | 0.39 | .74 | 1.70 | 1.61 | |

| Porcine No. | 7 | 14 | 21 | 28 | 35 | 42 | 46 days |
|---|---|---|---|---|---|---|---|
| 113 | <.3 | <.3 | .5 | 1.52 | -- | -- | 3.0 |
| 114 | <.3 | .3 | .6 | 1.35 | -- | -- | 3.0 |

*vaccinated with purified $VP_3$.

J.  Serology of animals vaccinated with fusion protein from pFM3 [G.]

Serum samples from 36 bovine (four groups of 9 each) inoculated with vaccine prepared from chromatographically purified fusion protein from pFM3, were collected on day 14, 21, 35, 56, 84, 112, 126 and 146 post vaccination. Each of the four groups was vaccinated with a different amount of protein. Group 1 received 10 µg on day 1 and 10 µg on day 112; Group 2 received 50 µg on day 1 and 50 µg on day 112; Group 3 received 250 µg on day 1; and Group 4 received 1250 µg on day 1. The serum samples were measured for the amount of neutralizing antibody induced using the Skinner suckling mouse assay. The results are shown in Figure 6 and give the median $\log_{10}$ $PD_{50}$ for each of the cattle groups. These results indicate that a significant titer of antibody is induced, and this level lasts several months.

K. Serology of animals vaccinated with fusion protein from pFMG ($O_1$ Campos Brazil/58)

Samples of protein from the pFMG ($O_1$ Campos) plasmid construction were prepared by a method similar to that described for pFM3. These samples were formulated in two dosage forms, 100 µg and 500 µg and administered into guinea pigs on day 1 and day 14. These samples were compared to similar samples containing 0.2 percent SDS, and also compared to virion isolated $VP_3$ from $O_1$ Campos. Guinea pig serum taken on day 35 and 56 was tested for $O_1$ Campos neutralizing antibody in the Skinner suckling mouse assay. The results shown below in Table 7 indicate that a significant titer of antibody is induced and that the levels are comparable to that obtained with the natural $VP_3$ from $O_1$ Campos.

Table 7

| Vaccination of Guinea Pigs With $O_1$ Campos Antigenic Proteins | | | |
|---|---|---|---|
| $O_1$ fusion protein from E. coli | 35 | 56 | dpv |
| $O_1$    100 µg | 1.5 | 1.8 | |
| $O_1$    500 µg | 2.2 | 3.2 | |
| $O_1$    100 µg + SDS | <.3 | 1.9 | |
| $O_1$    500 µg + SDS | 2.0 | 2.6 | |
| $O_1$ VP$_3$ from virion | | | |
| $O_1$    100 µg | 1.6 | 2.4 | |
| $O_1$    100 µg + SDS | 1.5 | 2.3 | |

Titer is expressed as the $-\log_{10}$ of serum dilution that protects 50 percent of suckling mice. Animals were vaccinated on day 0 and day 14. Each titer represents serums from five guinea pigs pooled before assay.

L. Serology of animals vaccinated with fusion protein from pFMB1 ($A_{24}$ Cruzeiro Brazil/55)

Samples of protein from the pFMB1 ($A_{24}$ Cruzeiro) plasmid construction were prepared by a method similar to that described for pFM3. These samples were formulated in two dosage forms, 100 µg and 500 µg and administered into guinea pigs (single dose). These samples were compared to similar samples containing 0.2 percent SDS. Guinea pig serums taken on day 35 were tested for $A_{24}$ Cruzeiro neutralizing antibody in the Skinner suckling mouse assay. The results shown below in Table 8 indicate that a significant titer of antibody is induced.

Table 8

Vaccination of Guinea Pigs With $A_{24}$ Cruzeiro Antigenic Proteins

$A_{24}$ fusion protein from E. coli

|  |  | 35 | dpv |
|---|---|---|---|
| $A_{24}$ | 100 µg | 1.6 | |
| $A_{24}$ | 500 µg | 3.1 | |
| $A_{24}$ | 100 µg + SDS | 2.8 | |
| $A_{24}$ | 500 µg + SDS | 3.1 | |

Each titer represents serums from five guinea pigs pooled before assay.

M. Challenge of Immunity

The challenge tests were done according to McKercher et al., Arch. Ges. Virtsforsch. 20, 190-197 (1967) and Graves et al., Res. Vet. Sci. 9, 35-40 (1968). The immunity elicited by the vaccine was challenged by placing the eight vaccinated bovine in 2 isolated rooms, four to a room and two vaccinated porcine in a separate room. In these rooms, they were exposed to 2 nonvaccinated animals, one of which was inoculated intradermalingually with 10,000 mouse $LD_{50}$ units of a virulent homologous strain of foot and mouth disease virus. An animal was considered protected from the disease when no sign or visible lesions of FMDV appeared after 14 days. The non-vaccinated bovine inoculated with the infectious virus and normal contact control became infected and manifested signs of foot and mouth disease. The vaccinated animals did not show signs of infection, with the exception of one animal that showed a single foot lesion. One of the animals vaccinated with the $VP_3$ protein purified from the virion did not respond with a high neutralizing antibody titer and was not protected. The two nonvaccinated control porcine became infected and were febrile. The two vaccinated porcine were free of FMDV infection.

A serum sample from each animal, taken after 14 days was examined for VIA (viral infection associated antigen). This served as a confirmation of the results of the challenge test. None of the animals vaccinated with the LE'-VP$_3$ showed the presence of VIA antigen in their serums. All of the controls did as well as the nonprotected natural VP$_3$ vaccinated bovine. See Table 9.

Table 9

| Cattle | Antigen | Challenge of Immunity | | VIA |
| | | Mouth Lesions | Foot Lesions | |
|---|---|---|---|---|
| 1 | LE'-VP$_3$ | 0 | 0 | – |
| 2 | LE'-VP$_3$ | 0 | 0 | – |
| 3 | LE'-VP$_3$ | 0 | 0 | – |
| 4 | LE'-VP$_3$ | 0 | 0 | – |
| 5 | LE'-VP$_3$ | 0 | + | – |
| 6 | LE'-VP$_3$ | 0 | 0 | – |
| 7 | VP$_3$ | 0 | 0 | – |
| 8 | VP$_3$ | +++ | +++ | + |
| 9 | None | +++ | +++ | + |
| 10 | None | +++ | +++ | + |
| 11 | None | +++ | +++ | + |
| 12 | None | +++ | +++ | + |
| Swine | | | | |
| 13 | LE'-VP$_3$ | 0 | 0 | – |
| 14 | LE'-VP$_3$ | 0 | 0 | – |
| 15 | None | +++ | +++ | + |
| 16 | None | +++ | +++ | + |

The demonstration herein that the microbially produced Serotype A FMDV protein, namely, containing an antigenic determinant of, for example, subtype $A_{12}$119ab, confers immunogenic protection to inoculated animals later exposed to the same FMDV subtype, is predictive of like protection against other strains of FMDV.

In preferred embodiments, vaccines are produced by providing proteins, microbially produced as herein described, which are protective against various strains of FMDV, i.e. multi-valent vaccines. For example, multi-valent vaccines can be produced which protect against $O_{38}$ and $A_{23}$ strains, $O_{445}$ and $A_{87}$ strains, $O_{75}$ and $A_{380}$ and $C_{22}$ strains, $O_{91}$ and $A_7$ strains and $O_{64}$ and $A_{178}$ and $C_{91}$ strains. For this purpose, the respective protective proteins can be prepared separately and mixed or prepared in tandem in a single polyprotein as herein described.

## 0. Synthetic Peptides

A set of synthetic peptides have been chemically prepared based on the amino acid sequences of the various $VP_3$ proteins. These peptides may contain one or more antigenic regions of the $VP_3$ proteins. The peptides prepared are listed in Table 10. Studies concerning the antigenic and immunogenic properties of these peptides, of other peptides synthesized based on amino acid sequence data from other parts of the $VP_3$ protein, as well as these peptides chemically linked to carrier proteins, should show if these preparations induce type specific antibodies in guinea pigs or rabbits. Further studies should indicate if any virus neutralizing antibodies are induced and if such preparations can be used as vaccines to protect cattle, swine, goats, etc. from FMD infection.

Table 10


Synthetic Peptides from VP$_3$ Protein Sequence


VP$_3$ A$_{24}$

1. Thr-Ser-Lys-Tyr-Ala-Val-Gly-Gly-Ser-Gly-Arg-Arg-Gly-Asp-Met-Gly

2. Tyr-Ala-Val-Gly-Gly-Ser-Gly-Arg-Arg-Gly-Asp-Met-Gly

3. Gly-Ser-Gly-Arg-Arg-Gly-Asp-Met-Gly

4. Arg-Gly-Asp-Met-Gly

VP$_3$ A arg/79

5. Thr-Ser-Lys-Tyr-Thr-Val-Gly-Gly-Ser-Gly-Arg-Arg-Gly-Asp-Met-Gly

6. Tyr-Thr-Val-Gly-Gly-Ser-Gly-Arg-Arg-Gly-Asp-Met-Gly

VP$_3$ C$_3$

7. Thr-Thr-Tyr-Thr-Thr-Ser-Ala-Arg-Arg-Arg-Gly-Asp-Leu

8. Thr-Ser-Ala-Arg-Arg-Gly-Asp-Leu

9. Arg-Gly-Asp-Leu

VP$_3$ O$_1$

10. Cys-Arg-Tyr-Ser-Arg-Asn-Ala-Val-Pro-Asn-Arg-Arg-Gly-Asp-Leu

11. Asn-Ala-Val-Pro-Asn-Arg-Arg-Gly-Asp-Leu

VP$_3$ A$_{12}$

12. Thr-Asn-Lys-Tyr-Ser-Ala-Ser-Gly-Ser-Gly-Val-Arg-Gly-Asp-Phe-Gly-Ser

13. Ser-Ala-Ser-Gly-Ser-Gly-Val-Arg-Gly-Asp-Phe-Gly-Ser

14. Gly-Asp-Phe-Gly-Ser

Claims

1. An immunogenically active, microbially produced polypeptide comprising the amino acid sequence of at least one antigenic determinant of foot and mouth disease virus protein.

2. An immunogenically active, microbially produced polypeptide comprising the amino acid sequence of at least two antigenic determinants of foot and mouth disease virus protein from at least two different foot and mouth disease virus strains.

3. The polypeptide according to Claim 2 wherein said strains are selected from the group consisting of A, C, O, Asia-1, SAT 1, SAT 2 and SAT 3 types.

4. The polypeptide according to Claim 1, 2 or 3 fused with additional polypeptide derived from expression of additional DNA.

5. The polypeptide according to Claim 4 wherein said additional DNA is synthetically derived and is in functional reading phase with DNA of the polypeptide of foot and mouth disease virus protein.

6. The polypeptide according to Claim 4 wherein said additional DNA is synthetically derived and is in functional reading phase with DNA encoding microbial polypeptide and with DNA of the polypeptide of foot and mouth disease virus protein.

7. The polypeptide according to Claim 4, 5 or 6, wherein said additional DNA encodes ATG in reading phase

8.    The polypeptide according to any of Claims 1 to 7, directly expressed.

9.    The polypeptide according to any preceding Claim comprising amino acids 55 to 177 of the $VP_3$ foot and mouth disease virus protein.

10.   The polypeptide according to any preceding Claim comprising amino acids 1 to 210 of the $VP_3$ foot and mouth disease virus protein.

11.   The polypeptide according to Claim 4 comprising amino acids 7 to 210 of the $VP_3$ foot and mouth disease virus protein.

12.   The polypeptide according to Claim 2 comprising amino acids 1 to 210 of the $VP_3$ foot and mouth disease virus protein fused to amino acids 8 to 210 of the $VP_3$ foot and mouth disease virus protein.

13.   A DNA sequence comprising a sequence coding for a polypeptide according to any preceding Claim.

14.   A replicable microbial expression vehicle capable, in a transformant microorganism, of expressing a polypeptide according to any of Claims 1 to 12.

15.   A replicable microbial expression vehicle comprising a DNA sequence according to Claim 13.

16.   A plasmid selected from $pFM_1$, $pFM_2$, $pFM_3$, $pFM_{10}$, $pFM_{20}$, pFMB1, pFMC, pFMD, pFMF and pFMG.

17.   A microorganism transformed with a vehicle according to any of Claims 14 to 16.

18.   A microorganism according to Claim 17, which is a transformed E. coli strain.

19. A microbial culture comprising a transformant microorganism according to Claim 17 or 18.

20. A microbial culture capable of producing a polypeptide according to any of Claims 1 to 12.

21. The use of a polypeptide according to any of Claims 1 to 12 for conferring immunogenicity to foot and mouth disease in a susceptible animal or for preparing a vaccine useful therefor.

22. A vaccine for use in conferring immunogenicity to foot and mouth disease in a susceptible animal comprising a polypeptide according to any of Claims 1 to 12 and a suitable vehicle.

23. A vaccine against foot and mouth disease comprising an immunogenically active, microbially produced constituent of foot and mouth disease virus.

24. A vaccine according to Claim 23 wherein said constituent is a polypeptide comprising the amino acid sequence of at least one antigenic determinant of foot and mouth disease virus.

25. A vaccine according to Claim 24 comprising antigenic determinant(s) of one or more foot and mouth disease virus strains.

26. A vaccine according to Claim 23, 24 or 25 wherein the foot and mouth disease virus strain is selected from A, C, O, Asia-1, SAT 1, SAT 2, and SAT 3 types.

27. A process which comprises microbially producing a polypeptide comprising the amino acid sequence of at least one antigenic determinant of foot and mouth disease virus protein.

28. The use of a DNA sequence according to Claim 13 or of an expression vehicle according to any of Claims 14 to 16 or of a microorganism or culture according to any of Claims 17 to 20 in the preparation of a polypeptide comprising the amino acid sequence of at least one antigenic determinant of foot and mouth disease virus protein.

Fig.1 (part a )

Fig. 1 (part b)

FMDV A$_5$

Fig.1 (part c)

```
                 1                                    10                                   20                                   30
A12 (T465)   thr thr ala thr gly glu ser ala asp pro val thr thr thr val glu asn tyr gly gly glu thr gln val gln arg arg his his thr
             ACC ACC GCT ACC GGG GAG TCT GCA GAC CCT GTC ACC ACC ACC GTG GAG AAC TAC GGT GGT GAG ACA CAA GTC CAG AGA CGT CAC CAC ACG
A24 (B435)           thr gly glu ser ala asp pro val thr thr thr val glu asn tyr gly gly glu thr gln ile gln arg arg his his thr
             ::: ::: ::: ACC GGG GAA TCA GCA GAC CCG GTC ACC ACC ACC GTG GAG AAC TAC GGC GGT GAG ACA CAA ATC CAG AGA CGT CAC CAC ACG
A27 (C312)   thr thr val thr gly glu ser ala asp pro val thr thr thr val glu asn tyr gly gly glu thr gln val gln arg arg his his thr
             ACC ACT GTC ACT GGG GAG TCC GCA GAC CCT GTC ACA ACC ACC GTG GAG AAC TAC GGC GGT GAG ACA CAA GTC CAA AGA CGG CAC CAC ACG
A79 (F64)    thr thr ala thr gly glu ser ala asp pro val thr thr thr val glu asn tyr gly gly glu thr gln val gln arg arg tyr his thr
             ACT ACC GCC ACC GGG GAG TCA GCA GAC CCT GTT ACC ACC ACC GTG GAG AAC TAC GGC GGT GAG ACA CAG GTT CAG AGA CGC TAC CAC ACT
C3 (D250)    thr met thr gly glu ser ala asp pro val thr thr thr val glu asn tyr gly gly glu thr gln val gln arg arg his his thr
             ACT ATG ACC ACT GGT GAA TCT GCC GAC CCT GTT ACC ACT ACC GTT GAG AAC TAC GGA GGA GAG ACG CAA GTT CAA CGT CGT CAC CAC ACT
01c (G85)    thr thr ser ala gly glu ser ala asp pro val thr ala thr val glu asn tyr gly gly glu thr gln ile gln arg arg gln his thr
             ACC ACT TCT GCG GGC GAG TCA GCG GAT CCT GTC ACC GCC ACT GTT GAA AAC TAC GGT GGC GAA ACA CAG ATC CAG AGG CGC CAA CAC ACG
01k          thr thr ser ala gly glu ser ala asp pro val thr thr thr val glu asn tyr gly gly glu thr gln ile gln arg arg gln his thr
             ACC ACT TCT GCG GGC GAG TCA GCG GAT CCT GTC ACC ACC ACC GTT GAA AAC TAC GGT GGC GAA ACA CAG ATC CAG AGG CGC CAA CAC ACG

                 40                                   50                                   60
A12 (T465)   asp val ser phe ile met asp arg phe val lys ile lys ser leu asn pro thr his val ile asp leu    met gln thr his gln his
             GAC GTC AGT TTC ATC ATG GAC AGA TTT GTG AAG ATA AAA AGC TTG AAC CCC ACA CAC GTC ATT GAC CTC ::: ATG CAG ACC CAC CAA CAC
A24 (B435)   asp ile gly phe ile met asp arg phe val lys ile gln ser leu ser pro thr his val ile asp leu    met gln thr his gln his
             GAC ATT GGT TTC ATC ATG GAC AGA TTT GTG AAG ATC CAA AGC TTG AGC CCA ACA CAT GTC ATT GAC CTC ::: ATG CAG ACT CAC CAA CAC
A27 (C312)   asp val gly phe ile met asp arg phe val lys ile asn asn leu ser pro thr his val ile asp leu    met gln thr his gln his
             GAT GTC GGG TTC ATT ATG GAC AGA TTT GTG AAA ATA AAC AAT CTG AGT CCC ACA CAT GTC ATT GAC CTC ::: ATG CAG ACC CAC CAG CAC
A79 (F64)    asp ile gly phe ile met asp arg phe val lys ile lys asp val gln pro thr his val ile asp leu    met gln thr his gln tyr
             GAC ATC GGC TTC ATC ATG GAC AGG TTT GTG AAG ATT AAG GAC GTG CAA CCG ACG CAT GTC ATT GAC CTT ::: ATG CAG ACT CAC CAA TAC
C3 (D250)    asp val ala phe val leu asp arg phe val lys val pro val ser asp gly gln gln his thr leu asp val met gln val his lys asp
             GAC GTT GCC TTC GTT CTT GAC CGG TTT GTG AAG GTC CCT GTG TCG GAC GGA CAA CAA CAC ACA CTG GAC GTG ATG CAG GTA CAC AAG GAC
01c (G85)    asp val ser phe ile met asp arg phe val lys ile thr pro gln asn gln ile asn ile leu asp leu    met gln ile pro ser his
             GAC GTC TCG TTC ATC ATG GAC AGA TTT GTG AAA GTG ACA CCG CAA AAC CAA ATT AAC ATT TTG GAC CTC ::: ATG CAG ATT CCA TCA CAC
01k          asp val ser phe ile met asp arg phe val lys val thr pro gln asn gln ile asn ile leu asp leu    met gln ile pro ser his
             GAC GTC TCG TTC ATC ATG GAC AGA TTT GTG AAG GTG ACA CCG CAA AAC CAA ATT AAC ATT TTG GAC CTC ::: ATG CAG ATT CCA TCA CAC

                 70                                   80                                   90
A12 (T465)   gly leu val gly ala leu leu arg ala ala thr tyr tyr phe ser asp leu glu ile val val arg his asp gly asn leu thr trp val
             GGG CTG GTG GGT GCG TTG TTG CGT GCA GCC ACG TAC TAC TTC TCC GAC TTG GAG ATT GTT GTG CGG CAC GAT GGC AAT CTG ACC TGG GTG
A24 (B435)   gly leu val gly ala leu leu arg ala ala thr tyr tyr phe ser asp leu glu ile val val arg his glu gly asn leu thr trp val
             GGT CTG GTG GGT GCC TTG CTG CGT GCA GCC ACG TAC TAC TTT TCT GAC TTG GAA ATT GTT GTA CGG CAC GAA GGC AAT CTG ACC TGG GTG
A27 (C312)   gly leu val gly ala leu leu arg ala ala thr tyr tyr phe ser asp leu glu ile val val arg his asp gly asn leu thr trp val
             GGG TTG GTA GGT GCG CTG TTG CGT GCA GCC ACC TAC TAC TTC TCT GAC CTT GAG ATT GTT GTG CGC CAC GAC GGC AAC CTG ACT TGG GTG
A79 (F64)    gly leu val gly ala met leu arg ala ala thr tyr tyr phe ser asp leu glu ile val val arg his asp gly asn leu thr leu val
             GGC CTG GTG GGT GCA ATG CTG CGT GCA GCT ACG TAC TAC TTT TCT GAC TTG GAA ATT GTT GTA CGG CAC GAC GGC AAC CTG ACT TTG GTG
C3 (D250)    ser ile val gly ala leu leu arg ala ala thr tyr tyr phe ser asp leu glu ile ala val thr his thr gly lys leu thr trp val
             AGT ATC GTG GGA GCG CTT CTC CGC GCA GCC ACG TAC TAC TTC TCT GAT CTG GAA ATA GCG GTG ACC CAC ACC GGG AAG CTC ACC TGG GTG
01c (G85)    thr leu val gly ala leu leu arg ala ser thr tyr tyr phe ser asp leu glu ile ala val lys his glu gly asp leu thr trp val
             ACT TTG GTG GGA GCG CTC CTA CGC GCG TCC ACT TAC TAC TTC TCT GAC TTG GAG ATA GCA GTA AAA CAC GAG GGA GAC CTC ACC TGG GTT
01k          thr leu val gly ala leu leu arg ala ser thr tyr tyr phe ser asp leu glu ile ala val lys his glu gly asp leu thr trp val
             ACT TTG GTG GGA GCA CTC CTA CGC GCG TCC ACT TAC TAC TTC TCT GAC TTG GAG ATA GCA GTA AAA CAC GAG GGA GAC CTC ACC TGG GTT
```

Fig.2 (part a)

```
                                    100                                 110                                 120
A12 (T465)  pro asn gly ala pro glu ala ala leu ser asn thr gly asn pro thr ala tyr asn lys ala pro phe thr arg leu ala leu pro tyr
            CCC AAC GGT GCC CCC GAG GCA GCC CTG TCA AAC ACC GGC AAC CCC ACT GCC TAC AAC AAG GCA CCG TTC ACG AGG CTT GCT CTC CCT TAC
A24 (B435)  pro asn gly ala pro glu ser ala leu leu asn thr ser asn pro thr ala tyr asn lys ala pro phe thr arg leu ala leu pro tyr
            CCC AAC GGC GCC CCT GAA TCA GCC CTG TTG AAC ACC AGC AAC CCC ACT GCC TAC AAC AAG GCA CCA TTC ACG AGA CTC GCT CTC CCC TAC
A79 (F64)   pro asn gly ala pro glu ser ala leu asp asn thr gly asn pro thr ala tyr asn lys ala pro phe thr arg leu ala leu pro tyr
            CCC AAC GGC GCC CCT GAG TCA GCC CTA GAC AAC ACT GGC AAT CCC ACC GCC TAC AAC AAG GCA CCA TTC ACG AGA CTT GCT CTC CCT TAC
A27 (C312)  pro asn gly ala pro glu ala ala leu ser asn thr ser asn pro thr ala tyr asn lys ala pro phe thr arg leu ala leu pro tyr
            CCC AAC GGT GCC CCC GAA GCA GCC CTC TCA AAC ACC AGC AAC CCC ACT GCC TAC AAC AAG GCA CCA TTC ACG AGA CTC GCT CTC CCG TAC
C3 (D250)   pro asn gly ala pro val ser ala leu asp asn thr thr asn pro thr ala tyr his lys arg pro leu thr arg leu ala leu pro tyr
            CCC AAC GGT GCA CCG GTT TCT GCA CTT GAC AAC ACA ACC AAC CCC ACT GCA TAC CAC AAG AGA CCG CTG ACT CGA CTG GCT CTC CCA TAC
01c (G85)   pro asn gly ala pro glu lys ala leu asp asn thr thr asn pro thr ala tyr his lys ala pro leu thr arg leu ala leu pro tyr
            CCA AAT GGA GCG CCT GAA AAG GCG TTG GAC AAC ACC ACC AAC CCA ACT GCT TAC CAC AAG GCA CCA CTC ACC CGG CTT GCC CTG CCC TAC
01k         pro asn gly ala pro glu lys ala leu asp asn thr thr asn pro thr ala tyr his lys ala pro leu thr arg leu ala leu pro tyr
            CCA AAT GGA GCG CCC GAA AAG GCG TTG GAC AAC ACC ACC AAC CCA ACT GCT TAC CAC AAG GCA CCA CTC ACC CGG CTT GCC CTG CCC TAC


                                    130                                 140                                 150
A12 (T465)  thr ala pro his arg val leu ala thr val tyr asn gly thr asn lys tyr ser ala ser gly ser gly     val arg gly asp phe gly
            ACT GCG CCA CAC CGC GTG TTG GCA ACT GTG TAC AAC GGG ACG AAC AAG TAC TCC GCG AGC GGT TCG GGA ::: GTG CGA GGC GAT TTT GGG
A24 (B435)  thr ala pro his arg val ser ala thr val tyr asn gly thr ser lys tyr ala val gly gly ser gly     arg arg gly asp met gly
            ACT GCG CCG CAC CGT GTG TCG GCA ACA GTG TAC AAC GGG ACG AGT AAG TAT GCT GTG GGT GGT TCA GGC ::: AGA CGT GGC GAC ATG GGG
A27 (C312)  thr ala pro his arg val leu ala thr val tyr asn phe thr asn lys tyr ser asn gly gly gln         arg ala gly asp met gly
            ACC GCG CCA CAC CGT GTG TTG GCA ACC GTG TAC AAC TTT ACG AAC AAG TAC TCC AAC GGT GGC CAG ::: ::: AGG GCA GGT GAC ATG GGG
A79 (F64)   thr ala pro his arg val leu ala thr val tyr asn gly thr ser lys tyr thr val gly gly ser gly     arg arg gly asp met gly
            ACG GCA CCA CAC CGT GTG CTG GCA ACA GTG TAC AAC GGG ACA AGC AAA TAC ACC GTG GGT GGT TCA GGC ::: AGG CGT GGT GAC ATG GGG
C3 (D250)   thr ala pro his arg val leu ala thr thr tyr thr gly thr thr thr tyr thr thr ser ala             arg arg gly asp leu val
            ACC GCG CCA CAC CGC GTG TTG GCC ACG ACG TAC ACT GGT ACA ACA ACC TAC ACT ACC AGT GCA ::: ::: ::: CGT AGA GGA GAT TTG GTC
01c (G85)   thr ala pro his arg val leu ala thr val tyr asn gly glu cys arg tyr ser arg asn ala val pro asn val arg gly asp leu gln
            ACC GCG CCC CAC CGC GTG TTG GCA ACC GTG TAC AAC GGT GAG TGC AGG TAC AGC AGA AAT GCT GTG CCC AAC GTG AGA GGT GAC CTT CAG
01k         thr ala pro his arg val leu ala thr val tyr asn gly glu cys arg tyr asn arg asn ala val pro asn leu arg gly asp leu gln
            ACT GCG CCC CAC CGC GTG TTG GCA ACC GTG TAC AAC GGT GAG TGC AGG TAC AAC AGA AAT GCT GTG CCC AAC TTG AGA GGT GAC CTT CAG


                                    160                                 170                                 180
A12 (T465)  ser leu ala pro arg val ala arg gln leu pro ala ser phe asn tyr gly ala ile lys ala glu thr ile his glu leu leu val arg
            TCT CTC GCG CCG CGA GTC GCG AGA CAA CTT CCT GCT TCT TTC AAC TAC GGT GCA ATT AAG GCC GAG ACC ATC CAC GAG CTT CTC GTG CGC
A24 (B435)  thr leu ala ala arg val val lys gln leu pro ala ser phe asn tyr gly ala ile lys ala asp ala ile his glu leu leu val arg
            ACT CTC GCG GCG CGA GTC GTG AAA CAG CTT CCT GCT TCA TTT AAC TAC GGT GCA ATC AAG GCC GAC GCC ATC CAC GAA CTT CTC GTG CGC
A27 (C312)  ser leu ala ala arg val ala lys gln leu pro ala ser phe asn tyr gly ala ile lys ala gln thr ile his glu leu leu val arg
            TCA CTT GCG GCA CGG GTC GCA AAA CAG CTT CCT GCT TCT TTC AAC TAC GGT GCA ATC AAG GCC CAG ACC ATC CAG GAG CTT CTC GTG CGC
A79 (F64)   ser leu ala ala arg val ala lys gln leu pro ala ser phe asn tyr gly ala ile lys ala thr ala ile his glu leu ile val arg
            TCC CTC GCG GCA CGA GTC GCG AAA CAA CTT CCT GCT TCA TTC AAC TAT GGT GCA ATT AAG GCC ACC GCC ATC CAC GAG CTC ATC CTG CGC
C3 (D250)   his leu ala ala ala his ala arg his leu pro thr ser phe asn phe gly ala val lys ala glu lys val thr glu leu leu val arg
            CAT TTG GCG GCA GCA CAC GCT CGG CAC TTG CCG ACG TCG TTC AAC TTT GGT GCA GTT AAA GCA GAA AAA GTC ACT GAG CTG CTG GTG CGC
01c (G85)   val leu ala gln lys val ala arg thr leu pro thr ser phe asn tyr gly ala ile lys ala thr arg val thr glu leu leu tyr arg
            GTG TTG GCT CAA AAG GTG GCA CGG ACG CTG CCT ACC TCC TTC AAC TAC GGT GCC ATC AAA GCG ACC CGG GTC ACC GAG TTG CTT TAC CGG
01k         val leu ala gln lys val ala arg thr leu pro thr ser phe asn tyr gly ala ile lys ala thr arg val thr glu leu leu tyr arg
            GTG TTG GCT CAA AAG GTG GCA CGG ACG CTG CCT ACC TCC TTC AAC TAC GGT GCC ATC AAA GCG ACC CGG GTC ACC GAG TTG CTT TAC CGG
```

**Fig.2 (part b)**

```
                                            190                                      200                                          210
A12 (T465)  met lys arg ala glu leu tyr cys pro arg pro leu leu ala ile glu val ser ser gln asp arg his lys gln lys ile ile ala pro
            ATG AAA CGG GCT GAG CTC TAC TGC CCC AGG CCA CTG CTG GCA ATA GAG GTG TCT TCG CAA GAC AGG CAC AAG CAG AAG ATC ATT GCA CCC
A24 (B435)  met lys arg ala glu leu tyr cys pro arg pro leu leu ala ile glu val ser ser gln asp arg his lys gln lys ile ile ala pro
            ATG AAA CGG GCC GAG CTC TAC TGC CCC AGA CCG CTG CTG GCA ATA GAG GTG TCT TCG CAA GAC AGG CAC AAG CAA AAG ATC ATT GCA CCA
A27 (C312)  met lys arg ala glu leu tyr cys pro arg pro leu leu ala ile glu val ser ser gln asp arg his lys gln lys ile ile ala pro
            ATG AAA AGG GCG GAG CTC TAC TGC CCC AGA CCA CTG TTG GCA ATA GAG GTG TCT TCG CAA GAC AGG CAC AAG CAG AAG ATC ATT GCG CCC
A79 (F64)   met lys arg ala glu leu tyr cys pro arg pro leu leu ala val glu val ser ser gln asp arg his lys gln lys ile ile ala pro
            ATG AAA CGG GCC GAA CTC TAC TGT CCC AGG CCA CTC TTG GCG GTG GAA GTG TCG TCA CAA GAT CGG CAC AAA CAG AAG ATC ATT GCA CCT
C3 (D250)   met lys arg ala glu leu tyr cys pro arg pro ile pro pro ile arg pro thr     gly asp arg his lys gln ser phe ile ala ser
            ATG AAG CGT GCA GAA CTC TAT TGC CCC AGG CCG ATT CCT CCG ATT CGG CCA ACG ::: GGC GAC AGA CAC AAG CAA TCG TTT ATC GCG TCG
O1c (G85)   met lys arg ala glu thr tyr cys pro arg pro leu leu ala ile his pro thr     glu ala arg his lys gln lys ile val ala pro
            ATG AAG AGG GCC GAA ACA TAC TGT CCA AGG CCC TTG CTG GCA ATC CAC CCA ACT ::: GAA GCC AGA CAC AAA CAG AAA ATT GTG GCA CCG
O1k         met lys arg ala glu thr tyr cys pro arg pro leu leu ala ile his pro thr     glu ala arg his lys gln lys ile val ala pro
            ATG AAG AGG GCC GAA ACA TAC TGT CCA AGG CCC TTG CTG GCA ATC CAC CCA ACT ::: GAA GCC AGA CAC AAA CAG AAA ATT GTG GCA CCG


                            220
A12 (T465)  gly lys gln     leu asn phe asp leu leu lys leu ala
            GGA AAA CAG ::: CTG AAC TTT GAC TTA CTC AAG TTG GCA
A24 (B435)  ala lys gln leu leu asn phe asp leu leu lys leu ala
            GCA AAG CAG CTT CTG AAT TTT GAC CTG CTC AAG TTG GCC
A27 (C312)  ala lys gln leu leu asn
            GCA AAG CAG TTG TTG AAT
A79 (F64)   ala lys gln pro leu
            GCA AAA CAG CCT TTG AA
C3 (D250)   ala lys gln pro ser asn leu
            GCG AAA CAG CCA TCA AAC CTT G
O1c (G85)   val lys gln thr leu
            GTG AAA CAG ACT TTG
O1k         val lys gln thr leu asn phe asp leu leu lys leu ala
            GTG AAA CAG ACT TTG AAT TTT GAC CTT CTC AAG TTG GCG
```

**Fig. 2 (part c)**

Fig.3

0068693

**Fig.4**

Fig. 5

PERCENT OF MAXIMUM BOUND (y-axis)

PICOMOLES OF VP$_3$ OR LE'-VP$_3$ ADDED (x-axis)

LE'-VP$_3$

VP$_3$

0068693

Fig.6